(19) Europäisches Patentamt
European Patent Office
Office européen des brevets

(11) Veröffentlichungsnummer: **0 065 488**
**B1**

(12) # EUROPÄISCHE PATENTSCHRIFT

(45) Veröffentlichungstag der Patentschrift: **14.08.85**

(51) Int. Cl.⁴: **C 07 D 205/08**, C 07 D 417/12

(21) Anmeldenummer: **82810199.8**

(22) Anmeldetag: **13.05.82**

(54) **Verfahren zur Herstellung von 4-Thio-azetidinon-Verbindungen.**

(30) Priorität: **19.05.81 CH 3245/81**

(43) Veröffentlichungstag der Anmeldung: **24.11.82 Patentblatt 82/47**

(45) Bekanntmachung des Hinweises auf die Patenterteilung: **14.08.85 Patentblatt 85/33**

(84) Benannte Vertragsstaaten: **BE CH DE FR GB IT LI NL SE**

(56) Entgegenhaltungen:
**DE - A - 2 352 198**
**DE - A - 2 606 278**
**DE - A - 2 613 641**

(73) Patentinhaber: **CIBA-GEIGY AG, Postfach, CH-4002 Basel (CH)**

(72) Erfinder: **Sedelmeier, Gottfried, Dr., St. Gallusstrasse 10, D-7801 Ehrenkirchen-Norsingen (DE)**

EP 0 065 488 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung betrifft ein neues Verfahren zur Herstellung von 4-Thio-azetidinon-Verbindungen, die als Ausgangsstoffe zur Herstellung von antibiotisch wirksamen 3-Cephemsäure-Derivaten verwendet werden können. Neue Zwischenprodukte sind ebenfalls Gegenstand der Erfindung.

Die Herstellung von 4-Thio-azetidinonen durch Umsetzung von Penicillansäure-1-oxiden mit Mercaptanen und gegebenenfalls anschliessender Umsetzung mit Schwermetall-, vor allem Silbersulfinaten, ist bereits in der Deutschen Offenlegungsschrift 2 506 330 beschrieben.

In der vorliegenden Erfindung wird ein Alternativverfahren zur Herstellung dieser synthetisch wertvollen Verbindungen aus leicht zugänglichen Ausgangssubstanzen vorgeschlagen. Der Vorteil des neuen Verfahrens liegt darin, dass die gewünschten Azetidinone in guten Ausbeuten zugänglich sind, dass auf die Verwendung teurer Silbersalze verzichtet werden kann und dass sich das erfindungsgemässe Verfahren zur Durchführung als sogenanntes Eintopfverfahren eignet.

Die Erfindung betrifft ein Verfahren zur Herstellung von 4-Thio-azetidinon-Verbindungen der Formel

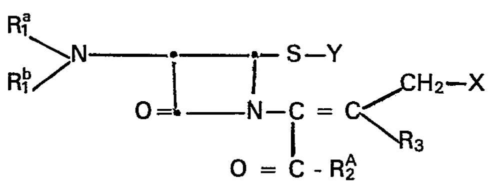

(I)

worin $R_1^a$ eine Aminoschutzgruppe und $R_1^b$ Wasserstoff oder einen Acylrest Ac bedeuten, oder worin $R_1^a$ und $R_1^b$ zusammen einen bivalenten Acylrest bilden, $R_2^A$ eine verätherte Hydroxygruppe ist, $R_3$ Methyl, Hydroxy, veräthertes oder verestertes Hydroxy, Cyano, tertiäres Amino oder Nitro ist, X Wasserstoff oder Halogen darstellt, und Y eine Gruppe $-SR_4$ bzw. $-SO_2-R_5$ ist, worin $R_4$ einen gegebenenfalls substituierten Heteroarylrest oder den Acylrest einer organischen Carbon- oder Thiocarbonsäure und $R_5$ einen gegebenenfalls substituierten Kohlenwasserstoffrest bedeutet, oder, wenn $R_3$ Hydroxy oder Methyl ist, Tautomeren davon, dadurch gekennzeichnet, dass man eine Verbindung der Formel

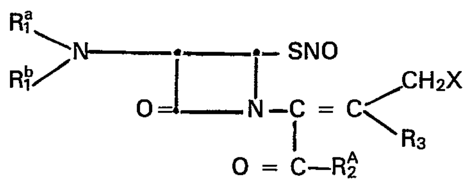

(III)

oder, wenn $R_3$ Hydroxy oder Methyl ist, ein Tautomeres davon mit einem Nitrosierungsmittel und einer Verbindung der Formel H-Y (IV) behandelt.

Wenn $R_3$ Hydroxy oder Methyl bedeutet, können die Verbindungen der vorliegenden Erfindung auch als Tautomere vorliegen. In solchen Tautomeren ist die Doppelbindung des Azetidinonstickstoff-Substituenten $\beta,\gamma$-ständig zur Carbonylgruppe des Restes $-C(=O)-R_2^A$, in der Form

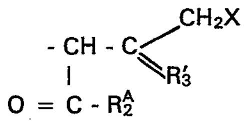

($R_3'$: Oxo oder Methylen), angeordnet. Die Verbindungen der vorliegenden Erfindung können aber auch als Gemisch der beiden Isomeren vorliegen.

In der nachfolgenden Beschreibung der Erfindung bedeutet der Ausdruck «Nieder» in Gruppen wie Niederalkyl, Niederalkylen, Niederalkoxy, Niederalkanoyl und dergleichen, dass die entsprechenden Gruppen, sofern nicht ausdrücklich anders definiert, bis zu 7, bevorzugt bis zu 4 C-Atome enthalten.

In den Verbindungen der Formeln (I) und (II) haben die Symbole $R_1^a$, $R_1^a$, $R_2^A$, $R_3$, X und Y bzw. $R_4$ und $R_5$ beispielsweise die folgenden Bedeutungen:

Eine Aminoschutzgruppe $R_1^a$ ist eine durch Wasserstoff ersetzbare Gruppe, in erster Linie eine Acylgruppe Ac, ferner eine Triarylmethylgruppe.

Eine Acylgruppe Ac ist in erster Linie der Acylrest einer organischen Säure, vorzugsweise mit bis zu 18, und in erster Linie mit bis zu 10 Kohlenstoffatomen, insbesondere der Acylrest einer gegebenenfalls substituierten aliphatischen, cycloaliphatischen, cycloaliphatisch-aliphatischen, aromatischen, araliphatischen, heterocyclischen oder heterocyclisch-aliphatischen Carbonsäuren.

Eine solche Acylgruppe ist in erster Linie ein Rest der Formel

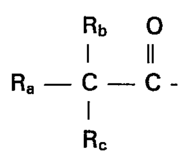

(IA)

worin (1) $R_a$ einen gegebenenfalls substituierten carbocyclischen Arylrest, z.B. entsprechendes Phenyl, einen gegebenenfalls substituierten, vorzugsweise ungesättigten cycloaliphatischen Kohlenwasserstoffrest, z.B. entsprechendes Cyclohexadienyl oder Cyclohexenyl, oder einen gegebenenfalls substituierten heterocyclischen Arylrest, z.B. entsprechendes Thienyl, Furyl oder Thiazolyl, $R_b$ Wasserstoff und $R_c$ Wasserstoff, geschütztes Amino oder gegebenenfalls geschütztes Hydroxy, Carboxyl oder Sulfo darstellen, oder worin (2) $R_a$ geschütztes $\omega$-Amino-$\omega$-carboxyniederalkyl, z.B. geschütztes 3-Amino-3-carboxy-propyl, Cyano, veräthertes Hydroxy oder Mercapto, wie gegebenenfalls substituiertes Phenyloxy, ferner Phenylthio oder Pyridylthio, oder einen gegebenenfalls substituierten, über ein Ringstickstoffatom verknüpften ungesättigten heterocyclischen Rest, z.B. entsprechendes Tetrazolyl, und $R_b$ und $R_c$ Wasserstoff bedeuten, oder worin (3) $R_a$ einen gegebenenfalls substituierten carbocyclischen Arylrest, z.B. entsprechendes Phenyl, oder einen gegebenenfalls substituierten heterocyclischen Arylrest, z.B. entsprechendes Thienyl, Furyl oder Thiazolyl, und $R_b$ und $R_c$ zusammen vorzugsweise O-substituiertes Hydroxyimino bedeuten.

Cyclohexadienyl ist insbesondere 1,4-Cyclohexadienyl, während Cyclohexenyl in erster Linie 1--Cyclohexenyl ist.

Thienyl ist vorzugsweise 2-, ferner 3-Thienyl, Furyl bedeutet insbesondere 2-Furyl, Thiazolyl ist insbesondere 4-Thiazolyl, während Pyridylthio z.B. 4-Pyridylthio, und Tetrazolyl z.B. 1-Tetrazolyl darstellen.

Substituenten einer Phenyl- oder Phenyloxygruppe $R_a$ können in irgendeiner Stellung vorhanden sein und sind unter anderem aliphatische Kohlenwasserstoffreste, wie gegebenenfalls substituiertes Niederalkyl, z.B. substituiertes Aminomethyl, gegebenenfalls funktionell abgewandeltes, wie veräthertes oder verestertes Hydroxy, substituiertes Amino oder Nitro, das z.B. in der Phenyloxygruppe in 2- oder 4-Stellung sein kann.

Substituenten einer Cyclohexadienyl- oder Cyclohexenylgruppe, sowie einer Thienyl- oder Furylgruppe $R_a$ sind z.B. gegebenenfalls substituiertes Niederalkyl, wie substituiertes Aminomethyl, wobei sich ein solcher Substituent in erster Linie in 2-Stellung eines 1,4-Cyclohexadienyl oder 1-Cyclohexenylrestes oder in 5-Stellung eines 2-Thienyl- oder 2-Furylrestes befindet. Substituiertes Thiazolyl ist insbesondere 2-Amino-4-thiazolyl, worin die Aminogruppe geschützt und/oder durch Niederalkyl substituiert ist.

Niederalkyl ist z.B. Methyl, Äthyl, Propyl, Isopropyl, Butyl oder tert.-Butyl. Substituiertes Aminomethyl ist in erster Linie geschütztes und gegebenenfalls zusätzlich durch Niederalkyl substituiertes Aminomethyl, z.B. geschütztes Methylaminomethyl, während veräthertes Hydroxy z.B. Niederalkoxy, wie Methoxy oder Äthoxy, und verestertes Hydroxy z.B. Niederalkanoyloxy, wie Acetyloxy oder Pivaloyloxy, Aroyloxy, z.B. Benzoyloxy, Carbamoyloxy oder Halogen, z.B. Chlor oder Brom, und substituiertes Amino z.B. geschütztes, gegebenenfalls zusätzlich durch Niederalkyl substituiertes Amino, z.B. geschütztes Methylamino, oder Niederalkylsulfonylamino, z.B. Methylsulfonylamino, sein kann.

O-substituiertes Hydroxyimino ist insbesondere Niederalkoxyimino, z.B. Methoxyimino oder Äthoxyimino, ferner Phenyloxyimino oder Phenylniederalkoxyimino, z.B. Benzyloxyimino, wobei solche Gruppen vorzugsweise in der syn-Form vorliegen.

Eine Triarylmethylgruppe $R_1^a$ ist in erster Linie eine Triphenylmethylgruppe.

Ein durch die Reste $R_1^a$ und $R_1^b$ zusammen gebildeter bivalenter Acylrest ist z.B. der Diacylrest einer organischen Dicarbonsäure, vorzugsweise mit bis zu 18 Kohlenstoffatomen, in erster Linie einer aliphatischen oder aromatischen Dicarbonsäure, z.B. der Acylrest einer Niederalkan- oder Niederalkendicarbonsäure, wie Succinyl, oder einer o-Arylendicarbonsäure, wie Phthaloyl. Ferner kommt in Betracht der Acylrest einer in α-Stellung vorzugsweise substituierten, z.B. einen aromatischen oder heterocyclischen Rest enthaltenden, α-Aminoessigsäure, worin die Aminogruppe über einen, vorzugsweise substituierten, z.B. Niederalkyl-, wie Methylgruppen, enthaltenden Methylenrest mit dem Stickstoffatom verbunden ist, z.B. ein insbesondere in 2-Stellung substituierter, z.B. gegebenenfalls substituiertes Phenyl oder Thienyl enthaltender, und in 4-Stellung gegebenenfalls durch Niederalkyl, wie Methyl, mono- oder disubstituierter 1-Oxo-3-aza-1,4-butylenrest, z.B. 4,4-Dimethyl-2-phenyl-1-oxo-3-aza--1,4-butylen.

Bevorzugt steht $R_1^a$ für Phenylacetyl oder Phenyloxyacetyl und $R_1^b$ für Wasserstoff.

Verätherte Hydroxygruppen $R_2^A$ sind durch aliphatische, cycloaliphatische, cycloaliphatisch-aliphatische, aromatische oder araliphatische Reste, insbesondere gegebenenfalls substituierte Kohlenwasserstoffreste dieser Art, sowie heterocyclische oder heterocyclisch-aliphatische Reste mit vorzugsweise bis zu 18 Kohlenstoffatomen verätherte Hydroxygruppen. Eine solche Gruppe ist z.B. 2-Halogen-niederalkoxy, worin Halogen vorzugsweise ein Atomgewicht von über 19 hat, z.B. 2,2,2-Trichloräthoxy oder 2-Jodäthoxy, ferner das leicht in letztere überführbare 2-Chloräthoxy oder 2-Bromäthoxy, oder 2-Niederalkylsulfonylniederalkoxy, z.B. 2-Methylsulfonyläthoxy. Die Gruppe $R_2^A$ ist ferner eine durch gegebenenfalls substituierte Kohlenwasserstoffreste, insbesondere gesättigte aliphatische oder aromatische Kohlenwasserstoffreste, wie Niederalkyl, z.B. Methyl, und/oder Phenyl polysubstituierte oder eine durch einen ungesättigten aliphatischen Kohlenwasserstoffrest, wie Niederalkenyl, z.B. Vinyl oder Allyl, durch eine Elektronen-abgebende Substituenten aufweisende carbocyclische Arylgruppe oder eine Sauerstoff oder Schwefel als Ringglied aufweisende heterocyclische Gruppe aromatischen Charakters monosubstituierte Methoxygruppe, wie tert.-Niederalkoxy, z.B. tert.-Butoxy- oder tert.--Pentoxy, gegebenenfalls substituiertes Diphenylmethoxy, z.B. Diphenylmethoxy oder 4,4'-Dimethoxydiphenyl-methoxy, Niederalkenyloxy, insbesondere 2-Niederalkenyloxy, z.B. Allyloxy, Niederalkoxy-phenylniederalkoxy, z.B. Niederalkoxy-benzyloxy, wie Methoxybenzyloxy (wobei Methoxy in erster Linie in 3-, 4- und/oder 5-Stellung steht), in erster Linie 3- oder 4-Methoxybenzyloxy oder 3,4--Dimethoxybenzyloxy, oder vor allem Nitrobenzyloxy, z.B. 2- oder 4-Nitrobenzyloxy, oder 4,5-Dimethoxy-2-nitro-benzyloxy, bzw. Furfuryloxy, wie 2-Furfuryloxy. $R_2^A$ kann auch 2-Oxo- oder 2-Thiacycloalkoxy oder -cycloalkenyloxy mit 5-7 Ringgliedern, wie 2-Tetrahydrofuryloxy, 2-Tetrahydropyranyloxy oder 2,3-Dihydro-2-pyranyloxy, oder Arylcarbonylmethoxy, worin Aryl insbesondere für eine gegebenenfalls substituierte Phenylgruppe steht, z.B. Phenacyloxy, oder gegebenenfalls substituiertes Phenyloxy, beispielsweise Nitrophenyloxy, z.B. 4-Nitrophenyloxy oder 2,4-Dinitrophenyloxy, oder Polyhalogenphenyloxy, z.B. Pentachlorphenyloxy sein. $R_2^A$ kann aber auch unverzweigtes Niederalkoxy, z.B. Methoxy oder Äthoxy, sein.

In Resten $R_3$ steht veräthertes Hydroxy insbesondere für Niederalkoxy, z.B. Methoxy oder Äthoxy, oder Arylniederalkoxy, z.B. Benzyloxy. Verestertes Hydroxy ist vor allem durch Niederalkancarbonsäuren, organische Sulfonsäuren, salpetrige Säure, Phosphin- oder Phosphorsäuren oder Halogenwasserstoffsäuren verestertes Hydroxy und bedeutet z.B. Niederalkanoyloxy, z.B. Formyloxy oder Acetyloxy, Sulfonyloxy, wie Methan- oder gegebenenfalls substituiertes Benzolsulfonyloxy, insbesondere p-Brom- oder p-Toluolsulfonyloxy, Nitrito, Phosphinoyloxy, z.B. Diphenylphosphinoyloxy, Phosphoryloxy, z.B. Diäthoxyphosphoryloxy, oder Halogen, z.B. Chlor oder Brom.

Tertiäres Amino $R_3$ ist beispielsweise eine Gruppe der Formel - $N(R_3^a)(R_3^b)$. Als Substituenten $R_3^a$ und $R_3^b$, die gleich oder verschieden sein können, kommen insbesondere aliphatische und cycloaliphatische Kohlenwasserstoffreste in Frage, die etwa bis zu 18, insbesondere bis zu 12 und bevorzugt bis zu 7 C-Atome enthalten. Aliphatische Kohlenwasserstoffreste sind beispielsweise gegebenenfalls substituierte, z.B. durch Niederalkoxy, wie Methoxy, Niederalkylthio, wie Methylthio, Cycloalkyl, wie Cyclohexyl, Aryl, wie Phenyl oder Heteroaryl, wie Thienyl, substituierte Niederalkylgruppen, z.B. Methyl, Äthyl, Propyl, Isopropyl, Butyl, Isobutyl, Pentyl, Hexyl, 2-Äthoxyäthyl, 2-Methylthioäthyl, Cyclohexylmethyl, Benzyl oder Thienylmethyl. Cycloaliphatische Kohlenwasserstoffreste sind beispielsweise gegebenenfalls substituierte, z.B. durch Niederalkyl, wie Methyl, Niederalkoxy, wie Methoxy, Niederalkylthio, wie Methylthio, Cycloalkyl, wie Cyclohexyl, Aryl, wie Phenyl oder Heteroaryl, wie Furyl, substituierte Cycloalkylgruppen, wie gegebenenfalls wie angegeben substituiertes Cyclopropyl, Cyclopentyl, Cyclohexyl oder Cycloheptyl. $R_3^a$ und $R_3^b$ kann auch zusammen für Niederalkylen, z.B. 1,4-Butylen oder 1,5-Pentylen, oder für durch Sauerstoff, Schwefel oder gegebenenfalls niederalkylierten, z.B. methylierten Stickstoff, unterbrochenes Niederalkylen, z.B. 3-Aza-, 3-Methyl-3-aza- oder 3-Oxapentylen stehen. Geeignete tertiäre Aminogruppen - $N(R_3^a)(R_3^b)$ sind beispielsweise Dimethylamino, Diäthylamino, Dicyclohexylamino, 1-Pyrrolidinyl, 4-Morpholinyl oder 4-Methyl-1-piperazinyl.

$R_3$ steht insbesondere für Hydroxy, Methyl, Methoxy, Acetoxy, Methansulfonyloxy, Nitrito, 1-Pyrrolidinyl oder 1-Morpholinyl.

X ist als Halogen insbesondere Chlor oder Brom.

In einer Gruppe - $SR_4$ ist $R_4$ z.B. ein gegebenenfalls substituierter Heteroaryl-Rest mit bis zu 15, bevorzugt bis zu 9 Kohlenstoffatomen, und mindestens einem Ringstickstoffatom und gegebenenfalls einem weiteren Ringhetero-, wie Sauerstoff- oder Schwefelatom, welcher mit einem seiner Ringkohlenstoffatome an die Thiogruppe - S - gebunden ist. Reste $R_4$ sind monocyclisch oder bicyclisch und können beispielsweise durch Niederalkyl, wie Methyl oder Äthyl, Niederalkoxy, wie Methoxy oder Äthoxy, Halogen, wie Fluor, Chlor oder Brom, oder Aryl, wie Phenyl, substituiert sein.

Reste $R_4$ sind z.B. fünfgliedrige Heteroaryl-Reste, wie Thiadiazolyl, Thiatriazolyl, Oxadiazolyl oder Oxatriazolyl, insbesondere aber Diazolyl, Oxazolyl und Thiazolyl und in erster Linie die entsprechenden Benzo-Derivate, wie Benzdiazolyl, Benzoxazolyl oder Benzthiazolyl, wobei ein substituierbares Ringstickstoffatom z.B. durch Niederalkyl, wie Methyl, substituiert sein kann. Ferner kommen sechsgliedrige Heteroaryl-Reste, z.B. Chinolyl, in Frage. Repräsentativ für solche Gruppen $R_4$ sind z.B. 1-Methyl-imidazol-2-yl, 1,3-Thiazol-2-yl, 1,3,4-Thiadiazol-2-yl, 1,3,4,5-Thiatriazol-2-yl, 1,3-Oxazol-2-yl, 1,3,4-Oxadiazol-2-yl, 1,3,4,5-Oxatriazol-2-yl, 1-Methyl-benzimidazol-2-yl, Benzoxazol-2-yl und insbesondere Benzthiazol-2-yl, ferner Chinolyl.

Weitere Gruppen $R_4$ sind Acylreste organischer Carbon- oder Thiocarbonsäuren, wie gegebenenfalls substituierte aliphatische, cycloaliphatische, araliphatische oder aromatische Acyl- oder Thioacylgruppen mit bis zu 18, vorzugsweise bis zu 10 Kohlenstoffatomen, wie Niederalkanoyl, z.B. Acetyl oder Propionyl, Niederthioalkanoyl, z.B. Thioacetyl oder Thiopropionyl, Cycloalkanoyl, z.B. Cyclohexanoyl, Cycloalkanthiocarbonyl, z.B. Cyclohexanthiocarbonyl, Benzoyl, Thiobenzoyl, Naphthoyl, Naphthylthiocarbonyl, heterocyclisches Carbonyl oder Thiocarbonyl, wie 2-, 3- oder 4-Pyridylcarbonyl, 2-oder 3-Thenoyl, 2- oder 3-Furoyl, 2-, 3- oder 4-Pyridylthiocarbonyl, 2- oder 3-Thiothenoyl, 2- oder 3-Thiofuroyl, oder entsprechende, beispielsweise durch Niederalkyl, wie Methyl, Halogen, wie Fluor oder Chlor, Niederalkoxy, wie Methoxy, Aryl, wie Phenyl, Aryloxy, wie Phenyloxy, mono- oder polysubstituierte Acyl- oder Thioacylgruppen.

Ein gegebenenfalls substituierter Kohlenwasserstoffrest $R_5$ in Gruppen - $SO_2$ - $R_5$ ist insbesondere ein entsprechender aliphatischer, cycloaliphatischer, araliphatischer oder aromatischer Kohlenwasserstoffrest mit bis zu 18, vorzugsweise bis zu 10 Kohlenstoffatomen. Geeignete Reste $R_5$ sind beispielsweise gegebenenfalls substituierte, wie durch Niederalkoxy, z.B. Methoxy, Halogen, z.B. Fluor, Chlor oder Brom, Aryl, z.B. Phenyl, Aryloxy, z.B. Phenyloxy, mono- oder polysubstituierte Alkyl-, insbesondere Niederalkyl-, z.B. Methyl-, Äthyl- oder Butylgruppen, Alkenyl-, z.B. Allyl- oder Butenylgruppen, Cycloalkyl-, z.B. Cyclopentyl- oder Cyclohexylgruppen, oder gegebenenfalls durch Niederalkyl, z.B. Methyl, Niederalkoxy, z.B. Methoxy, Halogen, z.B. Fluor, Chlor oder Brom, Aryl, z.B. Phenyl, Aryloxy, z.B. Phenyloxy, oder Nitro mono- oder polysubstituierte Naphthyl- oder insbesondere Phenylgruppen, beispielsweise Phenyl, 2-, 3- oder bevorzugt 4-Tolyl, 2-, 3- oder bevorzugt 4-Methoxyphenyl, 2-, 3- oder 4-Chlorphenyl, 4-Biphenylyl, 4-Phenyloxyphenyl, 4-Nitrophenyl oder 1- oder 2-Naphthyl.

Bevorzugte Reste $R_4$ sind z.B. 1,3-Oxazol-2-yl, 1,3-Thiazol-2-yl, Benzoxazol-2-yl sowie insbesondere Benzthiazol-2-yl. Bevorzugte Reste $R_5$ sind z.B. unsubstituiertes oder durch Methyl, Methoxy, Brom oder Nitro substituiertes Phenyl, insbesondere p-Tolyl.

Geschützte Hydroxy-, Amino-, Carboxyl- oder Sulfogruppen in Acylresten der Formel (IA) sind solche, die in der Penicillin- und Cephalosporin-Chemie üblich sind, und die leicht in freie Hydroxy-, Amino-, Carboxyl- oder Sulfogruppen überführt werden können, ohne dass dabei das Molekülgerüst zerstört wird oder andere unerwünschte Nebenreaktionen stattfinden.

Schutzgruppen dieser Art, sowie ihre Abspaltung sind beispielsweise beschrieben in «Protective Groups in Organic Chemistry», Plenum Press, London, New York, 1973, ferner in «The Peptides», Vol. I, Schröder and Lübke, Academic Press, London, New York, 1965, sowie in Houben-Weyl, «Methoden der organischen Chemie», 4. Auflage, Band 15/1, Georg Thieme Verlag, Stuttgart, 1974.

Aminogruppen können z.B. durch Acylreste geschützt sein, wobei ein Acylrest in erster Linie ein durch Reduktion, z.B. durch Behandeln mit einem chemischen Reduktionsmittel oder mit katalytisch aktiviertem Wasserstoff, oder durch Solvolyse, z.B. durch Behandeln mit einer geeigneten Säure, ferner auch mittels Bestrahlen, abspaltbarer Acylrest eines Halbesters der Kohlensäure ist, wie ein, vorzugsweise am ersten Kohlenstoffatom der veresternden Gruppe mehrfach verzweigter und/oder durch Aryl, z.B. gegebenenfalls, wie durch Niederalkoxy, z.B. Methoxy, und/oder Nitro substituiertes Phenyl oder Biphenylyl, oder durch Arylcarbonyl, insbesondere Benzoyl, substituierter Niederalkoxycarbonylrest, z.B. tert.-Butoxycarbonyl, tert.-Pentoxycarbonyl, Diphenylmethoxycarbonyl, 2-(4-Biphenylyl)-2-propoxycarbonyl, Benzyloxycarbonyl, 4-Nitrobenzyloxycarbonyl, 2-Nitro-4,5-dimethoxy-benzyloxycarbonyl oder Phenacyloxycarbonyl, oder ein am zweiten Kohlenstoffatom der veresternden Gruppe durch Halogen substituierter Niederalkoxycarbonylrest, 2,2,2-Trichloräthoxycarbonyl oder 2-Jodäthoxycarbonyl, ferner polycyclisches Cycloalkoxycarbonyl, z.B. Adamantyloxycarbonyl.

Eine Aminogruppe kann ferner durch einen Arylmethyl-, wie Polyarylmethylrest, z.B. durch Trityl, eine 2-Carbonyl-vinyl-gruppierung, wie eine 1-Niederalkoxycarbonyl-1-propen-2-ylgruppe, z.B. 1-Methoxycarbonyl-1-propen-2-yl, eine Arylthio- oder Arylniederalkylthiogruppe, z.B. 2-Nitrophenylthio oder Pentachlorphenylthio, ferner Tritylthio, eine Phosphinoylgruppe, z.B. Diphenylphosphinoyl, oder eine Sulfonylgruppe, wie eine Niederalkansulfonyl-, z.B. Methansulfonyl-, oder Arensulfonyl-, z.B. p-Toluolsulfonylgruppe, geschützt sein.

Hydroxyschutzgruppen sind z.B. Acylreste, insbesondere einer der im Zusammenhang mit einer geschützten Aminogruppe genannten Acylreste von Kohlensäurehalbestern, ferner leicht abspaltbare 2-Oxa- oder 2-Thia-aliphatische oder -cycloaliphatische Kohlenwasserstoffreste, in erster Linie 1-Niederalkoxy-niederalkyl oder 1-Niederalkylthio-niederalkyl, z.B. 1-Methoxyäthyl, 1-Äthoxy-äthyl, 1-Methylthio-äthyl oder 1-Äthylthio-äthyl, oder 2-Oxa- oder 2-Thiacycloniederalkyl mit 5-7 Ringatomen, z.B. 2-Tetrahydrofuryl oder 2-Tetrahydropyranyl, oder die entsprechenden Thiaanaloge, sowie leicht abspaltbare, gegebenenfalls substituierte $\alpha$-Phenylniederalkylreste, wie gegebenenfalls substituiertes Benzyl oder Diphenylmethyl, wobei als Substituenten der Phenylreste z.B. Halogen, wie Chlor, Niederalkoxy, wie Methoxy, und/oder Nitro in Frage kommen.

Eine geschützte Carboxyl- oder Sulfogruppe ist in erster Linie eine mit einem aliphatischen, cycloaliphatischen, cycloaliphatisch-aliphatischen, aromatischen oder araliphatischen Alkohol, wie einen Niederalkanol, veresterte Carboxyl- oder Sulfogruppe. In einer Carboxyl- oder Sulfogruppe kann die Hydroxygruppe beispielsweise durch einen Alkohol der Formel $R_2^A$-H verestert sein.

Die Erfindung betrifft insbesondere ein Verfahren zur Herstellung von Verbindungen der Formel I, worin $R_1^a$ eine Acylgruppe der Formel IA darstellt, worin (1) $R_a$ gegebenenfalls durch Hydroxy, geschütztes Hydroxy, Niederalkyl, Niederalkoxy, Niederalkanoyloxy, Aroyloxy, Halogen, Niederalkylsulfonylamino, Nitro oder substituiertes Aminomethyl substituiertes Phenyl oder durch Niederalkyl oder substituiertes Aminomethyl substituiertes Thienyl, Furyl, Cyclohexadienyl oder Cyclohexenyl oder durch Diniederalkylamino oder geschütztes Amino substituiertes Thiazolyl darstellt, $R_b$ Wasserstoff und $R_c$ Wasserstoff, gegebenenfalls geschütztes Hydroxy, geschütztes Amino, geschütztes Carboxyl oder geschütztes Sulfo darstellt, oder worin (2) $R_a$ geschütztes 3-Amino-3-carboxy-propyl, Cyano, 1-Tetrazolyl, gegebenenfalls durch Hydroxy, geschütztes Hydroxy, Niederalkyl, Niederalkoxy, Niederalkanoyloxy, Aroyloxy, Halogen, Niederalkylsulfonylamino, Nitro oder substituiertes Aminomethyl substituiertes Phenyloxy, oder Pyridylthio und $R_b$ und $R_c$ Wasserstoff darstellen, oder worin (3) $R_a$ gegebenenfalls durch Hydroxy, geschütztes Hydroxy, Niederalkyl, Niederalkoxy, Niederalkanoyloxy, Aroyloxy, Halogen, Niederalkylsulfonylamino, Nitro oder substituiertes Aminomethyl substituiertes Phenyl oder durch Niederalkyl oder substituiertes Aminomethyl substituiertes Thienyl oder Furyl oder durch Diniederalkylamino oder geschütztes Amino substituiertes Thiazolyl darstellt, und $R_b$ und $R_c$ zusammen syn-Niederalkoxyimino bedeuten, und $R_1^b$ Wasserstoff ist, $R_2^A$ Niederalkoxy, 2-Halogenniederalkoxy, 2-Niederalkenyloxy, durch gegebenenfalls durch Niederalkoxy und/oder Nitro substituiertes Phenyl mono- oder disubstituiertes Methoxy, Trityloxy oder gegebenenfalls durch Nitro oder Halogen substituiertes Phenyloxy ist, $R_3$ Methyl, Hydroxy, Niederalkoxy, Phenylniederalkoxy, Niederalkanoyloxy, Sulfonyloxy, Nitrito, Phosphinoyloxy, Phosphoryloxy, Halogen, Cyano, Nitro oder durch Niederalkyl, Niederalkoxyniederalkyl oder Cycloalkyl disubstituiertes oder durch Sauerstoff, Schwefel oder gegebenenfalls niederalkyliertes Stickstoff unterbrochenes Niederalkylen als Substituenten aufweisendes Amino bedeutet, X Wasserstoff oder Halogen bedeutet und Y für einen Rest $-SR_4$ oder $-SO_2-R_5$ steht, worin $R_4$ gegebenenfalls durch Niederalkyl substituiertes Thiadiazolyl, Oxathiazolyl, Diazolyl, Oxazolyl, Thiazolyl, Benzthiazolyl, Benzoxazolyl oder Benzthiazolyl bedeutet und $R_5$ Niederalkyl, Cycloalkyl, gegebenenfalls durch Niederalkyl, Niederalkoxy, Halogen, Phenyl, Phenyloxy oder Nitro substituiertes Phenyl oder Naphthyl ist, oder, wenn $R_3$ Hydroxy oder Methyl ist, Tautomeren davon, aus entsprechend substituierten Verbindungen der Formel III.

Die Erfindung betrifft vorzugsweise ein Verfahren zur Herstellung von Verbindungen der Formel I, worin $R_1^a$ eine Acylgruppe der Formel IA darstellt, worin $R_a$ unsubstituiertes oder durch geschütztes Hydroxy, z.B. tert.-Butoxycarbonyloxy oder 2-Tetrahydropyranyloxy, Niederalkoxy, z.B. Methoxy, oder geschütztes Aminomethyl, z.B. tert.-Butoxycarbonylaminomethyl, substituiertes Phenyl oder Phenyloxy, oder Thienyl, z.B. 2-Thienyl, Furyl, z.B. 2-Furyl, Cyclohexadienyl, z.B. 1,4-Cyclohexadienyl, oder geschütztes 3-Amino-3-carboxypropyl, z.B. 3-

-Benzoylamino-3-diphenylmethoxycarbonyl-propyl, und $R_b$ und $R_c$ Wasserstoff sind, und $R_1^b$ Wasserstoff darstellt, $R_2^A$ Niederalkoxy, z.B. Methoxy oder tert.-Butoxy, 2-Halogenniederalkoxy, z.B. 2-Jod- oder 2,2,2-Trichloräthoxy, 2-Niederalkenyloxy, z.B. Allyloxy, unsubstituiertes oder in 4-Stellung durch Nitro substituiertes Benzyloxy, unsubstituiertes oder in 4- und 4'-Stellung durch Methoxy disubstituiertes Diphenylmethoxy oder Trityloxy ist, $R_3$ Methyl, Niederalkoxy, z.B. Methoxy, Phenylniederalkoxy, z.B. Benzyloxy, Hydroxy, Sulfonyloxy, z.B. Methan- oder p-Toluolsulfonyloxy, Nitrito, Phosphinoyloxy, z.B. Diphenylphosphinoyloxy, Phosphoryloxy, z.B. Diäthoxyphosphoryloxy, Halogen, z.B. Brom, Cyano, Nitro, Diniederalkylamino, z.B. Dimethylamino, Dicycloalkylamino, z.B. Dicyclohexylamino, 1-Pyrrolidinyl, 4-Morpholinyl oder 4-Methyl-1-piperazinyl bedeutet, X Wasserstoff oder Halogen, z.B. Brom ist, und Y für Heteroarylthio, wie Oxazolyl-, z.B. 1,3-Oxazol-2-yl-, Thiazolyl-, z.B. 1,3-Thiazol-2-yl-, Benzoxazol-2-yl- oder Benzthiazol-2-ylthio, oder für unsubstituiertes oder durch Niederalkyl, z.B. Methyl, Niederalkoxy, z.B. Methoxy, Halogen, z.B. Brom, oder Nitro substituiertes Benzolsulfonyl steht, oder, wenn $R_3$ Hydroxy oder Methyl ist, Tautomeren davon, aus entsprechend substituierten Verbindungen der Formel III.

Die Erfindung betrifft in erster Linie ein Verfahren zur Herstellung von 3-Amino-4-thioazetidin-2-onen der Formel I, worin $R_1^a$ für einen Acetylrest der Formel IA steht, worin $R_a$ Phenyl, Thienyl, z.B. 2-Thienyl, oder Phenyloxy ist, und $R_b$ und $R_c$ Wasserstoff bedeuten, $R_1^b$ Wasserstoff ist, $R_2^A$ Niederalkoxy, z.B. tert.-Butoxy, 2-Halogenniederalkoxy, z.B. 2,2,2-Trichloräthoxy, gegebenenfalls in 4-Stellung durch Nitro substituiertes Benzyloxy, oder Diphenylmethoxy ist, X Wasserstoff oder Halogen, z.B. Brom, darstellt, $R_3$ Methyl, Hydroxy, Niederalkoxy, z.B. Methoxy, Sulfonyloxy, z.B. Methansulfonyloxy, Nitrito, Diniederalkylamino, z.B. Dimethylamino, 1-Pyrrolidinyl oder 1-Morpholinyl bedeutet und Y bicyclisches Heteroarylthio, z.B. Benzthiazol-2-ylthio, oder unsubstituiertes oder in 4-Stellung durch Niederalkyl, z.B. Methyl, substituiertes Benzolsulfonyl darstellt, oder, wenn $R_3$ Hydroxy oder Methyl ist, Tautomeren davon, aus entsprechend substituierten Verbindungen der Formel III.

Für das erfindungsgemässe Verfahren können anorganische oder organische Nitrosierungsmittel verwendet werden. Anorganische Nitrosierungsmittel sind beispielsweise salpetrige Säure und Anhydride derselben, z.B. das symmetrische Anhydrid Distickstofftrioxid, ferner Distickstofftetroxid sowie gemischte Anhydride mit Halogenwasserstoffsäuren, wie Nitrosyl-chlorid oder -bromid, ferner Nitrosyl-tetrafluoroborat und Nitrosyl-schwefelsäure. Als organische Nitrosierungsmittel sind insbesondere Niederalkylester der salpetrigen Säure geeignet, wobei der Niederalkylrest insbesondere Äthyl, Isopropyl, n-Butyl, n-Pentyl oder Isopentyl (Isoamyl) ist.

Die Verbindungen der Formel IV sind Mercaptane der Formel $R_4$ - SH(IVa) und Sulfinsäuren der Formel $R_5$ - $SO_2H$(IVb), worin $R_4$ und $R_5$ die angegebenen Bedeutungen haben.

Die Umsetzung der Thionitrite der Formel III mit den Verbindungen der Formel IV kann in Gegenwart zumindest stöchiometrischer Mengen eines der genannten Nitrosierungsmittel, wie salpetriger Säure, Distickstofftrioxid, Distickstofftetroxid oder eines Niederalkylnitrits, z.B. Isoamylnitrit, in einem inerten Lösungsmittel, beispielsweise in einem Niederalkanol, z.B. Äthanol, oder in einem chlorierten Kohlenwasserstoff, z.B. Methylenchlorid, Chloroform oder Tetrachloräthan, oder bei Verwendung von salpetriger Säure oder eines Niederalkylnitrits als Nitrosierungsmittel, auch in einem zweiphasigen Wasser-organischen Lösungsmittel-System erfolgen, wobei als organisches Lösungsmittel insbesondere chlorierte Kohlenwasserstoffe, z.B. Chloroform, Methylenchlorid oder Tetrachloräthan, Diniederalkyläther, z.B. Diäthyl- oder Di-n-butyläther, oder ein Kohlenwasserstoff, z.B. Dekalin, in Frage kommt. Die Reaktion wird in einem Temperaturbereich zwischen —70° und +50°C und bevorzugt bei etwa —10° bis etwa +40°C durchgeführt.

In einer bevorzugten Ausführungsform des Verfahrens setzt man Thionitrite der Formel III mit Verbindungen der Formel IVa bzw. IVb in Gegenwart von Isoamylnitrit als Nitrosierungsmittel im System Wasser-Chlorkohlenwasserstoff, z.B. -Methylenchlorid, bei etwa 20° bis 25°C um.

Die Herstellung der Ausgangsstoffe der Formel III kann unter solchen Bedingungen durchgeführt werden, dass man diese Stoffe aus dem Reaktionsgemisch isolieren und, z.B. mittels chromatographischer Methoden, reinigen kann, bevor ihre Weiterverarbeitung zu den Endprodukten der Formel I erfolgt. Vorzugsweise werden die Ausgangsstoffe der Formel III jedoch in situ hergestellt und ohne Isolierung aus dem Reaktionsgemisch weiter zu den Endprodukten der Formel I umgesetzt. Diese «Eintopfverfahren» stellen ebenfalls einen Gegenstand der Erfindung dar.

Die Erfindung betrifft daher ebenso Verfahren zur Herstellung von Verbindungen der Formel III

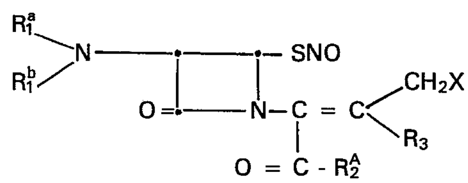

(III)

worin $R_1^a$ eine Aminoschutzgruppe und $R_1^b$ Wasserstoff oder einen Acylrest Ac bedeuten, oder worin $R_1^a$ und $R_1^b$ zusammen einen bivalenten Acylrest bilden, $R_2^A$ eine verätherte Hydroxygruppe ist, $R_3$ Methyl, Hydroxy, veräthertes oder verestertes Hydroxy, Cyano, tertiäres Amino oder Nitro ist und X Wasserstoff oder Halogen darstellt, oder, wenn $R_3$ Hydroxy oder Methyl ist, Tautomeren davon, dadurch gekennzeichnet, dass man

a) ein Mercapto-azetidinon der Formel

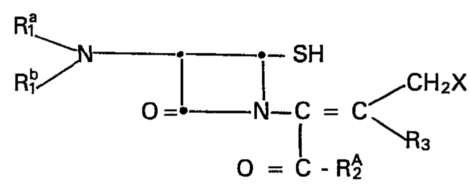

(II)

oder ein Salz oder, wenn $R_3$ Hydroxy oder Methyl ist, ein Tautomeres davon, mit einem Nitrosierungsmittel behandelt, oder dass man

b) zur Herstellung von 4-Nitrosothio-azetidinonen der Formel

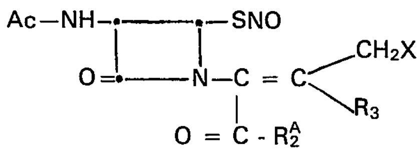

(III'),

worin Ac eine Acylgruppe bedeutet, und $R_2^A$, $R_3$ und X die unter Formel III angegebenen Bedeutungen haben, oder, wenn $R_3$ Hydroxy oder Methyl ist, einem Tautomeren davon, ein Diazabicyclohepten der Formel

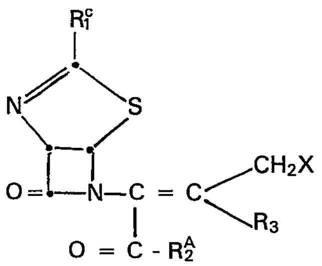

(V)

worin $R_1^c$ einen von der Acylgruppe Ac abgeleiteten, um die Carbonylgruppe verminderten Rest bedeutet, oder, wenn $R_3$ Hydroxy oder Methyl ist, ein Tautomeres davon, mit einem sauren Hydrolysemittel behandelt und ein erhaltenes Zwischenprodukt der Formel

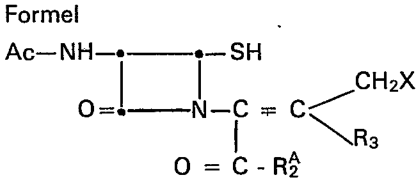

(II')

oder, wenn $R_3$ Hydroxy oder Methyl ist, ein Tautomeres davon, gegebenenfalls ohne Isolierung aus dem Reaktionsgemisch mit einem Nitrosierungsmittel behandelt, oder dass man

c) zur Herstellung von 4-Nitrosothio-azetidinonen der Formel

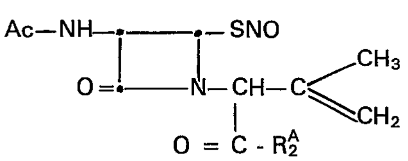

(III'')

ein Penicillin-1-oxid der Formel

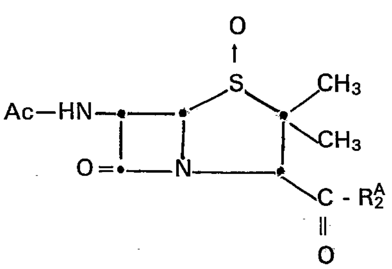

(VI),

worin Ac die unter Formel III' und $R_2^A$ die unter Formel III angegebene Bedeutung hat, mit einer Verbindung des 3wertigen Phosphors umsetzt, ein erhaltenes Zwischenprodukt der Formel

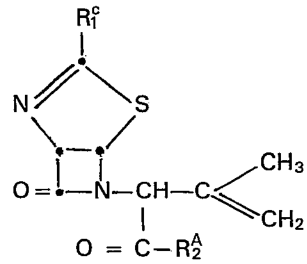

(V'),

worin $R_1^c$ die unter Formel V angegebene Bedeutung hat, gegebenenfalls ohne Isolierung aus dem Reaktionsgemisch mit einem sauren Hydrolysemittel behandelt und das erhaltene Zwischenprodukt der Formel

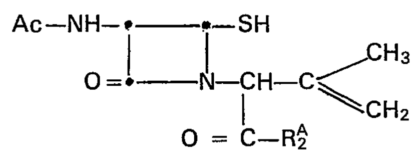

(II')

gegebenenfalls ohne Isolierung aus dem Reaktionsgemisch mit einem Nitrosierungsmittel behandelt.

Die Erfindung betrifft insbesondere die in den Beispielen genannten Verfahren zur Herstellung von Verbindungen der Formel III.

*Verfahren a)*

Salze von Verbindungen der Formel II sind beispielsweise Alkalimetall-, z.B. Natrium- oder Kalium-, Erdalkalimetall-, z.B. Calcium-, oder Schwermetall-, z.B. Silber- oder Quecksilbersalze.

Als Nitrosierungsmittel können anorganische oder organische Nitrosierungsmittel, wie die oben im Zusammenhang mit der Herstellung der Endprodukte der Formel I beschriebenen, verwendet werden.

Die Nitrosierung der Mercaptane der Formel II wird in einem gegenüber dem Nitrosierungsmittel inerten Lösungsmittel durchgeführt. Bei der Verwendung von salpetriger Säure, die vorteilhaft durch Einwirken einer Mineralsäure, z.B. Salz- oder Schwefelsäure, einer Niederalkansäure, z.B. Essigsäure, oder eines sauren Salzes, z.B. Kaliumhydrogen- oder Aluminiumsulfat, auf ein Alkalimetall-, z.B. Natriumnitrit, erzeugt wird, oder eines ionischen Nitrosylsalzes, z.B. Nitrosyl-tetrafluoroborat, arbeitet man vorzugsweise in wässriger Phase, wobei die Verbindungen der Formel II durch starkes Rühren in Suspension gehalten werden, oder in einem zweiphasigen System, das neben Wasser noch eine Lösungsmittelphase für die Verbindung der Formel II enthält, beispielsweise einen Kohlenwasserstoff, z.B. Benzol, Dekalin oder Hexan, einen chlorierten Kohlenwasserstoff, z.B. Methylenchlorid, Chloroform oder Tetrachloräthan, einen Diniederalkyläther, z.B. Diäthyl- oder Di-n-butyläther, oder einen Ester, z.B. Äthylacetat. Die Nitrosierung kann aber auch in homogener Lösung erfolgen, beispielsweise in Eisessig. Bei der Verwendung von Distickstofftrioxid, Nitrosylhalogeniden oder Niederalkylnitriten als Nitrosierungsmittel arbeitet man, wie beschrieben, in einem zweiphasigen Lösungsmittelsystem, oder in homogener organischer Phase, beispielsweise in einem der genannten Kohlenwasserstoffe, chlorierten Kohlenwasserstoffe oder Diniederalkyläther, oder in einem Niederalkanol, z.B. Äthanol oder n-Butanol, in 1,2-Dimethoxyäthan, Diäthylenglykolmonomethyläther oder in einem Säureamid, z.B. Dimethylformamid oder Hexamethylphosphorsäuretriamid. Die Umsetzung mit Nitrosylhalogeniden erfolgt gegebenenfalls in Gegenwart einer Base, wie eines Alkali- oder Erdalkalicarbonates, z.B. Natrium-, Kalium- oder Magnesiumcarbonat, oder vorzugsweise einer organischen Stickstoffbase, z.B. Pyridin, Chinolin, Dimethylanilin oder Triäthylamin, wobei die genannten Stickstoffbasen gleichzeitig als Lösungsmittel dienen können. Salze von Verbindungen der Formel II, z.B. Alkali-, wie Natriumsalze, können mit Nitrosylhalogeniden umgesetzt werden, wobei man vorteilhaft ein solches Lösungsmittel wählt, in dem ein sich bildendes anorganisches Salz, z.B. ein Alkalihalogenid, unlöslich ist und aus dem Reaktionsgemisch ausfällt. Hierzu eignen sich besonders die genannten Diniederalkyläther, chlorierten Kohlenwasserstoffe und Benzol. Falls notwendig, wird die Nitrosierung in einer Inertgas-, z.B. Stickstoffatmosphäre, durchgeführt.

Die Reaktionstemperatur liegt zwischen —70° und +50°C und bevorzugt zwischen —20° und +40°C.

Eine bevorzugte Ausführungsform stellt die Nitrosierung mit Isoamylnitrit im zweiphasigen Wasser-Halogenkohlenwasserstoff-, z.B. Wasser-Methylenchlorid-System, bei etwa 20° bis 25°C dar.

Das verfahrensgemäss erhältliche Thionitrit der Formel III kann gegebenenfalls ohne Isolierung aus dem Reaktionsgemisch weiter zu den Endprodukten der Formel I umgesetzt werden.

Dabei geht man von einer Verbidnung der Formel II aus und führt die Nitrosierung, vorzugsweise in Wasser-Chlorkohlenwasserstoff-, z.B. Methylenchlorid-Phase, mit mindestens 2 Moläquivalenten eines Nitrosierungsmittels, z.B. mit Isoamylnitrit, in Gegenwart einer Verbindung der Formel IVa bzw. IVb durch, wobei das intermediär gebildete Thionitrit der Formel III in situ zu den Endprodukten der Formel I weiterreagiert.

Die Ausgangsstoffe der Formel II sind bekannt bzw. können nach an sich bekannten Methoden hergestellt werden.

Die Ausgangsstoffe der Formel II, worin $R_1^b$ Wasserstoff ist, können beispielsweise hergestellt werden, indem man 4-Thia-2,6-diazabicyclo[3.2.0]-heptene der Formel

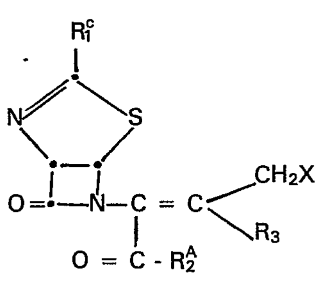

(V)

oder Tautomere davon sauer hydrolysiert, wie in der belgischen Patentschrift 838 656 und unter Verfahren b) nachstehend beschrieben.

Die Ausgangsstoffe der Formel II können auch hergestellt werden, indem man ein Azetidinon der Formel

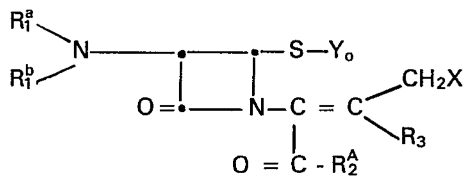

(VII)

worin $Y_0$ einen solvolytisch abspaltbaren Rest, z.B. die Trimethylsilyl-Gruppe, darstellt, oder, wenn $R_3$ Hydroxy oder Methyl ist, ein Tautomeres davon, solvolysiert, z.B. mit wässriger Mineralsäure, wie Salzsäure, hydrolysiert, oder mit Alkoholen, z.B. Methanol, solvolysiert. Die genannten Verbindungen der Formel VII sind erhältlich, indem man ein Mercaptan der Formel VIII (beschrieben in der deutschen Offenlegungsschrift 2 655 298)

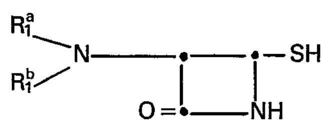

(VIII)

mit Trimethylsilylchlorid behandelt und in die entstandene Verbindung der Formel IX

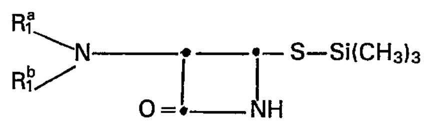

(IX)

durch Umsetzung mit einer Verbindung der Formel X

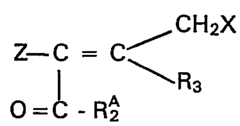

(X)

worin Z Halogen, z.B. Brom, ist, oder, wenn $R_3$ Hydroxy oder Methyl ist, einem Tautomeren davon, den Rest $-C(-COR_2^A)=C(R_3)(CH_2X)$ bzw. $-CH(-COR_2^A)-C(=R_3')(CH_2X)$ einführt. Man kann die Verbindungen der Formel IX aber auch mit einem Glyoxylsäureester der Formel $OHC-CO-R_2^A$ umsetzen, in dem entstandenen Addukt der Formel XI

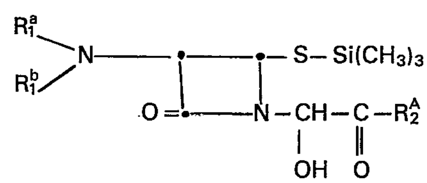

(XI)

die Hydroxygruppe mit Phosphortribromid gegen Brom austauschen und dann nach dem Wittig-Verfahren den Rest $-C(-COR_2^A)=C(R_3)(CH_2X)$ bzw. $-CH(-COR_2^A)-C(=R_3')(CH_2X)$ aufbauen.

*Verfahren b)*

Ein von der Acylgruppe Ac abgeleiteter, um die Carbonylgruppe verminderter Rest $R_1^c$ ist in erster Linie ein Rest der Formel - C($R_a$, $R_b$, $R_c$), worin $R_a$, $R_b$ und $R_c$ die oben angegebenen Bedeutungen haben. $R_1^c$ ist bevorzugt Benzyl oder Phenyloxymethyl.

Ein saures Hydrolysemittel ist insbesondere eine wässrige Säure. Vorzugsweise werden anorganische Säuren verwendet, wie Halogenwasserstoffsäuren, z.B. Salzsäure, Schwefelsäure, Salpetersäure, Phosphorsäure, Tetrafluoroborsäure, Perchlorsäure oder Chlorsäure, organische Sulfonsäuren, wie Niederalkan-, z.B. Methansulfonsäure, gegebenenfalls durch Methyl oder Brom substituierte Benzolsulfonsäuren, z.B. p-Toluolsulfonsäure, Aralkylsulfonsäuren, z.B. Benzylsulfonsäure, oder $\alpha$-Halogenalkansulfonsäuren, z.B. Trifluormethansulfonsäure, $\alpha$-Halogencarbonsäuren, z.B. Trifluor- oder Dichloressigsäure, oder Polycarbonsäuren, z.B. Oxalsäure.

Das erfindungsgemässe Verfahren b) kann bevorzugt als «Eintopfverfahren» durchgeführt werden. Man kann das Verfahren aber auch auf der Stufe des Mercaptans der Formel II' abbrechen, das Produkt isolieren und dieses in die weiteren Reaktionsschritte einsetzen.

Die Hydrolyse der Diazabicycloheptene der Formel V erfolgt mit einer wässrigen Lösung einer der genannten Säuren. Man arbeitet in einem organischen Lösungsmittel, in dem sich die Säure und Wasser lösen, z.B. einem Niederalkanol, wie Äthanol, einem Niederalkanon, z.B. Aceton, einem Amid, z.B. Dimethylformamid, einem Sulfoxid, z.B. Dimethylsulfoxid oder in überschüssigem Wasser, oder unter zweiphasigen Bedingungen in einem Lösungsmittel, in dem sich die Ausgangsverbindung der Formel V löst, z.B. in einem halogenierten Kohlenwasserstoff, wie Methylenchlorid, einem Ester, wie Äthylacetat, oder einem Äther, wie Diäthyläther, oder in Gemischen dieser Lösungsmittel. Um Nebenreaktionen, wie Aufspaltung des Azetidinon-Ringes zu vermeiden, arbeitet man bei einer Reaktionstemperatur von etwa 10° bis 30°C, vorzugsweise bei Raumtemperatur.

Die weitere Umsetzung des entstandenen Mercaptans II' zu dem Thionitrit der Formel III', oder, im «Eintopfverfahren», zu den Endstoffen der Formel I, worin $R_1^a$ einen Acylrest Ac und $R_1^b$ Wasserstoff bedeutet und $R_2^A$, $R_3$ und Y die unter Formel I angegebenen Bedeutungen haben, erfolgt in gleicher Weise, wie unter Verfahren a) beschrieben. Dabei arbeitet man vorzugsweise in einem der aufgeführten zweiphasigen Lösungsmittel-Systeme mit einem Niederalkylnitrit oder salpetriger Säure als Nitrosierungsmittel.

In einer besonders bevorzugten Ausführungsform des erfindungsgemässen Verfahrens geht man von der Lösung einer Verbindung der Formel V in einem Chlorkohlenwasserstoff, z.B. Methylenchlorid, aus, hydrolysiert gegebenenfalls in Gegenwart eines zweiten organischen Lösungsmittels, das mit Wasser mischbar ist und als Lösungsvermittler dienen kann, z.B. Aceton, mit verdünnter Mineral-, z.B. Salz- oder Schwefelsäure, oder Sulfonsäure, z.B. p-Toluolsulfonsäure, und setzt das entstandene Azetidinon II' ohne Isolierung aus dem Reaktionsgemisch mit 2 Moläquivalenten eines Nitrosierungsmittels, z.B. Isoamylnitrit, in Gegenwart eines Mercaptans der Formel IVa oder einer Sulfinsäure der Formel IVb um, wobei das intermediär gebildete Thionitrit III' <u>in situ</u> zu den Endprodukten der Formel I weiterreagiert.

*Verfahren c)*

Eine Verbindung des dreiwertigen Phosphors ist vorzugsweise ein Triniederalkyl-, z.B. Trimethyl- oder Triäthylphosphit, oder ein Triaryl-, insbesondere Triphenylphosphin.

Das erfindungsgemässe Verfahren c) kann bevorzugt als «Eintopfverfahren» durchgeführt werden. Man kann das Verfahren aber auch auf einer beliebigen Verfahrensstufe abbrechen, das Produkt isolieren und dieses in die weiteren Reaktionsschritte einsetzen.

Die Überführung des Penicillin-1-oxids der Formel VI in das bicyclische Amid der Formel V' erfolgt in einem gegenüber den Reaktionsteilnehmern inerten Lösungsmittel, beispielsweise in einem aromatischen Kohlenwasserstoff, z.B. Benzol oder Toluol, oder in einem chlorierten Kohlenwasserstoff, z.B. Äthylenchlorid oder Tetrachlorkohlenstoff. Man arbeitet in Gegenwart eines milden, wasserentziehenden Mittels, beispielsweise Calciumchlorid, Kieselgel oder eines Molekularsiebs, oder unter destillativer, vorzugsweise azeotrop-destillativer Entfernung des Reaktionswassers. Die Reaktionstemperatur liegt bei etwa 70° bis 110°C, vorzugsweise bei der Siedetemperatur des verwendeten Lösungsmittels.

Die, gegebenenfalls nach Abfiltrieren des wasserentziehenden Mittels, entstandene Lösung des bicyclischen Amids der Formel V' kann direkt, ohne zusätzliche Isolierungsarbeiten, weiter umgesetzt werden. Man kann die Lösung aber auch konzentrieren und als Konzentrat weiterverwenden.

Die folgenden Reaktionsschritte — Überführung des entstandenen bicyclischen Amids der Formel V' über das Mercaptan der Formel II'' in das Thionitrit der Formel III'' und dessen weitere Überführung, gegebenenfalls ohne Isolierung aus dem Reaktionsgemisch, in den Endstoff der Formel

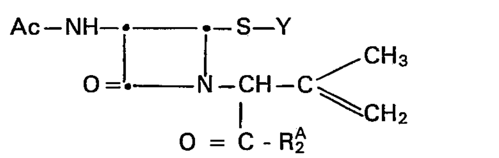

(I'),

erfolgt in gleicher Weise, wie unter Verfahren a) und b) beschrieben.

In einer besonders bevorzugten Ausführungsform des erfindungsgemässen Verfahrens geht man z.B. von der Lösung eines Penicillin-1-oxids der Formel VI in einem Chlorkohlenwasserstoff, z.B. Äthylenchlorid, aus, setzt mit einer Verbindung des dreiwertigen Phosphors und einem wasserentziehenden Mittel, z.B. mit Trimethylphosphit und mit einem Molekularsieb, um, filtriert das wasserentziehende Mittel ab, hydrolysiert das entstandene bicyclische Amid der Formel V', gegebenenfalls nach vorhergehender

Konzentration der Lösung, mit verdünnter Mineral-, z.B. Salzsäure, oder Sulfon-, z.B. p-Toluolsulfonsäure, und setzt das entstandene Azetidinon II'' ohne Isolierung aus dem Reaktionsgemisch mit 2 Moläquivalenten eines Nitrosierungsmittels, z.B. Isoamylnitrit, in Gegenwart eines Mercaptans der Formel IVa oder einer Sulfinsäure der Formel IVb um, wobei das intermediär gebildete Thionitrit III'' in situ zu den Endprodukten der Formel I' weiterreagiert.

Das Verfahren umfasst auch diejenigen Ausführungsformen, wonach Zwischenprodukte isoliert und die restlichen Verfahrensschritte mit diesen durchgeführt werden, die Ausgangsstoffe in situ hergestellt und ohne Isolierung verwendet werden oder das Verfahren auf irgendeiner Stufe abgebrochen wird; ferner können Ausgangsstoffe in Form von Derivaten verwendet oder während der Reaktion gebildet werden.

Vorzugsweise werden solche Ausgangsstoffe verwendet und die Reaktionsbedingungen so gewählt, dass man zu den eingangs als besonders bevorzugt aufgeführten Verbindungen gelangt.

Die Endprodukte der Formel I können, beispielsweise analog der deutschen Offenlegungsschrift 2 506 330, in antibiotisch wirksame 7β-Acylamino-3-cephem-4-carbonsäuren überführt werden.

Die Erfindung betrifft ausserdem die neuen Verbindungen der Formel

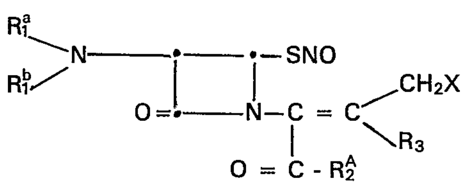

(III)

worin $R_1^a$, $R_1^b$, $R_2^A$, $R_3$ und X die unter Formel I angegebenen, insbesondere die bevorzugten Bedeutungen haben, und Tautomere davon.

Die folgenden Beispiele dienen zur Illustration der Erfindung und sollen sie in keiner Weise einschränken. Temperaturen werden in Celsius-Graden angegeben.

*Beispiel 1:*

*3-Methyl-2-(4-nitrosothio-2-oxo-3-phenoxyacetylamino-azetidin-1-yl)-3-butensäure-diphenylmethylester*

Zu einer Lösung von 516 mg 3-Methyl-2-(4-mercapto-2-oxo-3-phenoxyacetylamino-azetidin-1-yl)-3-butensäure-diphenylmethylester in 10 ml Methylenchlorid gibt man bei Raumtemperatur 0,5 ml wässriger 40%iger p-Toluolsulfonsäure und tropft nach 15 Minuten 234 mg (2 mMol) Isoamylnitrit in 2 ml Methylenchlorid hinzu. Nach 7 Stunden Rühren bei Raumtemperatur wird die dunkelrote Lösung dreimal mit je 10 ml Wasser gewaschen, anschliessend unter Lichtausschluss eingedampft und im Hochvakuum entgast. Man erhält 3-Methyl-2-(4-nitrosothio-2-oxo-3-phenoxyacetylamino-azetidin-1-yl)-3-butensäure-diphenylmethylester.

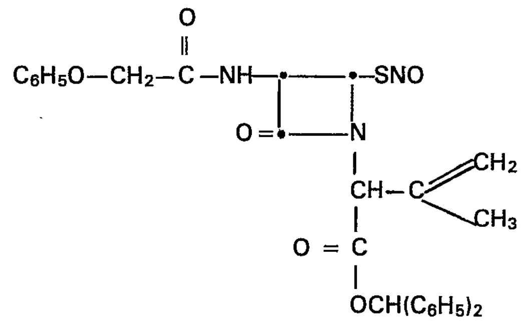

als rötlich glänzenden Schaum.

Dünnschichtchromatogramm in Toluol/Essigsäureäthylester (2:1) : $R_f$: 0,7 (olivgrüner Fleck); IR (in Methylenchlorid): Absorptionsbanden bei 1780, 1740, 1695, 1520, 1490 und 1230 $cm^{-1}$.

Das Ausgangsmaterial kann wie folgt hergestellt werden:

Man löst 500 mg (1 mMol) 3-Methyl-2-(3-phenoxymethyl-7-oxo-4-thia-2,6-diazabicyclo[3.2.0]-hept-2-en-6-yl)-3-butensäure-diphenylmethylester

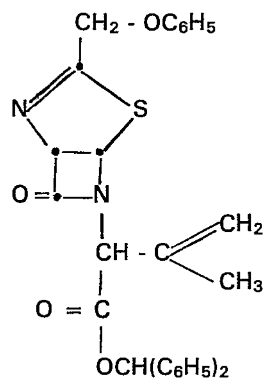

in 10 ml eines Gemisches aus Methylenchlorid/Aceton (1:1). Hierzu gibt man bei Raumtemperatur 1 ml 35%ige Perchlorsäure und rührt die klare Lösung 50 Minuten bei Raumtemperatur weiter. Anschliessend gibt man 50 ml Methylenchlorid und 50 ml Wasser hinzu, trennt die organische Phase ab und extrahiert noch zweimal mit je 30 ml Methylenchlorid. Nach Trocknen der vereinigten organischen Phasen über Natriumsulfat und Abdestillieren des Lösungsmittels wird aus Äther/Hexan (1:1) kristallisiert. Man erhält 3-Methyl-2-(4-mercapto-2-oxo-3-phenoxyacetylamino-azetidin-1-yl)-3-butensäure-diphenylmethylester vom Smp. 54-57°.

IR (in Methylenchlorid): Absorptionsbanden bei 3400, 3025, 2910, 1775, 1740, 1695, 1515, 1495, 1230, 1175 und 1155 $cm^{-1}$.

$^1H$-NMR ($CDCl_3$, 100 MHz): δ = 1,72 (m, 1H), 1,90 (s, 1H), 4,58 (s, 2H), 4,90-5,00 (m, 2H), 4,05-4,15 (m, 2H), 5,40-5,55 (m, 1H) ppm.

*Beispiel 2:*

*3-Methyl-2-[4-(4-methylphenylsulfonylthio)-2-oxo-3-phenoxyacetylamino-azetidin-1-yl]-3-butensäure-diphenylmethylester*

a) Zur Lösung von 3,0 g 3-Methyl-2-(3-phenoxymethyl-7-oxo-4-thia-2,6-diazabicyclo[3.2.0]hept-2-en-6-yl)-3-butensäure-diphenylmethylester in 30

ml Methylenchlorid gibt man bei Raumtemperatur 1,25 g 4-Toluolsulfinsäure und 0,5 g 40%ige 4--Toluolsulfonsäure. Nach 15 Minuten Rühren bei Raumtemperatur tropft man eine Lösung von 1,87 g Isoamylnitrit in 10 ml Methylenchlorid innerhalb von 30 Minuten zu. Die Lösung verfärbt sich von gelb über grün und orange nach dunkelrot. Man rührt weitere 16 Stunden bei Raumtemperatur, wonach sich die Lösung wieder nach gelbgrün entfärbt hat. Die Lösung wird mit gesättigter Bicarbonytlösung dreimal gewaschen, das Lösungsmittel und überschüssiges Isoamylnitrit sowie gebildeter Isoamylalkohol werden im Vakuum bzw. Hochvakuum abdestilliert und der entstandene 3-Methyl-2-[4--(4-methylphenylsulfonylthio)-2-oxo-3-phenoxyacetylamino-azetidin-1-yl]-3-butensäure-diphenylmethylester

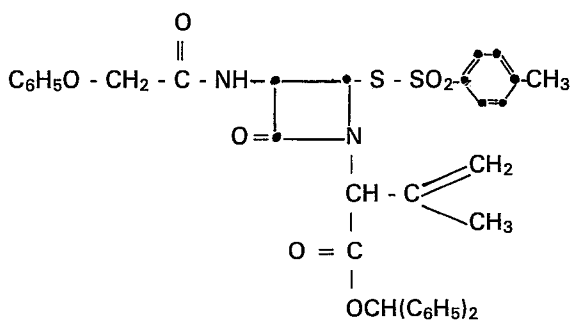

wird nach Auflösen in wenig Methylenchlorid mit Diäthyläther ausgefällt. $R_f$ in Toluol/Essigsäureäthylester (2:1), 0,55; IR (in Chloroform): Absorptionsbanden bei 1782, 1740, 1695, 1340 und 1150 $cm^{-1}$. Die gleiche Verbindung kann auch wie folgt erhalten werden:

b) Zur Lösung von 3,82 g (7 mMol) 3-Methyl-2--(4-mercapto-2-oxo-3-phenoxyacetylamino-azetidin-1-yl)-3-butensäure-diphenylmethylester in 10 ml Methylenchlorid tropft man innerhalb von 15 Minuten 1,56 g (10 mMol) p-Toluolsulfinsäure in 20 ml Methylenchlorid und anschliessend 890 mg (10 mMol) Isopropylnitrit in 5 ml Methylenchlorid hinzu. Nach 15 stündigem Rühren bei Raumtemperatur unter Lichtausschluss hat sich die ursprünglich rote Lösung nach hellgelb entfärbt. Die Aufarbeitung des Reaktionsgemisches erfolgt in gleicher Weise, wie unter a) beschrieben.

c) Man löst 5,32 g (10 mMol) 6-Phenoxyacetylamino-penicillansäure-diphenylmethylester-1-oxid in 50 ml Äthylenchlorid, setzt bei Raumtemperatur 5 g Molekularsieb und 1,85 g Trimethylphosphit zu und erhitzt 24 Stunden am Rückfluss. Anschliessend wird filtriert, dreimal mit je 25 ml Wasser gewaschen und im Vakuum auf 35 ml eingedampft. Zu der entstandenen Lösung von 3-Methyl-2-(3-phenoxymethyl-7-oxo-4-thia-2,6-diazabicyclo[3.2.0]hept-2--en-6-yl)-3-butensäure-diphenylmethylester gibt man 2,1 g (13,5 mMol) p-Toluolsulfinsäure und 1 ml 30%ige wässrige p-Toluolsulfonsäure und rührt bei Raumtemperatur. Anschliessend tropft man innerhalb von 15 Minuten eine Lösung von 3,04 g (26 mMol) Isoamylnitrit in 5 ml Äthylenchlorid zu, wobei die Innentemperatur von 22° auf 27° ansteigt. Die Lösung verfärbt sich über hell- und dunkelgrün nach dunkelrot. Man rührt die Lösung weitere 18 Stunden unter Lichtausschluss bei Raumtemperatur, wonach sich die Lösung wieder nach gelb entfärbt hat. Die Aufarbeitung erfolgt in der gleichen Weise, wie unter a) beschrieben.

*Beispiel 3:*

*2-[4-(Benzthiazol-2-yldithio)-2-oxo-3-phenoxyacetylamino-azetidin-1-yl]-3-methyl-3-butensäure-diphenylmethylester*

Zur Lösung von 4,0 g (8 mMol) 3-Methyl-2-(3--phenoxymethyl-7-oxo-4-thia-2,6-diazabicyclo-[3.2.0]hept-2-en-6-yl)-3-butensäure-diphenylmethylester in 12 ml Methylenchlorid gibt man 400 mg 40%ige wässrige p-Toluolsulfonsäure und 1,55 g (17 mMol) Isopropylnitrit und rührt 6 Stunden bei Raumtemperatur unter Lichtausschluss. Anschliessend tropft man eine Lösung von 1,67 g (10 mMol) 2-Mercapto-benzthiazol in 30 ml Essigsäureäthylester innerhalb von 30 Minuten hinzu und rührt weitere 12 Stunden bei Raumtemperatur. Nach dieser Zeit zeigt das Dünnschichtchromatogramm nahezu vollständigen Umsatz zur Titelverbindung an. Die Aufarbeitung erfolgt in analoger Weise, wie in Beispiel 2a) beschrieben. Die Titelverbindung hat einen Smp. von 140-141°.

*Beispiel 4:*

*3-Methyl-2-(4-nitrosothio-2-oxo-3-phenylacetylamino-azetidin-1-yl)-3-butensäure-diphenylmethylester*

In analoger Weise, wie in Beispiel 1 beschrieben, erhält man ausgehend von 3-Methyl-2-(4-mercapto--2-oxo-3-phenylacetylamino-azetidin-1-yl)-3-butensäure-diphenylmethylester die Titelverbindung als rötliches Öl, Dünnschichtchromatogramm in Toluol/-Essigsäureäthylester (2:1) : $R_f$: 0,45.

Das Ausgangsmaterial kann in analoger Weise, wie in Beispiel 2c) beschrieben, dargestellt werden, wobei man 6-Phenylacetylamino-penicillansäure-diphenylmethylester-1-oxid mit Trimethylphosphit und einem Molekularsieb in Äthylenchlorid umsetzt und den entstandenen 2-(3-Benzyl-7-oxo-thia-2,6--diazabicyclo[3.2.0]hept-2-en-6-yl)-3-methyl-3-butensäure-diphenylmethylester mit wässriger p-Toluolsulfonsäure hydrolysiert.

*Beispiel 5:*

In analoger Weise, wie in Beispiel 2c beschrieben, kann man die folgenden Verbindungen herstellen:

3-Methyl-2-[4-(4-methylphenylsulfonylthio)-2--oxo-3-phenylacetylamino-azetidin-1-yl]-3-butensäure-diphenylmethylester, Smp. 75°, Dünnschichtchromatogramm in Toluol/Essigsäureäthylester (1:1) : $R_f$: 0,47, und

2-[4-(Benzthiazol-2-yldithio)-2-oxo-3-phenylacetylamino-azetidin-1-yl]-3-methyl-3-butensäure-diphenylmethylester, Smp. 134-136°, Dünnschichtchromatogramm in Toluol/Essigsäureäthylester (1:1) : $R_f$: 0,52.

**Patentansprüche**

1. Verfahren zur Herstellung von 4-Thio-azetidinon-Verbindungen der Formel

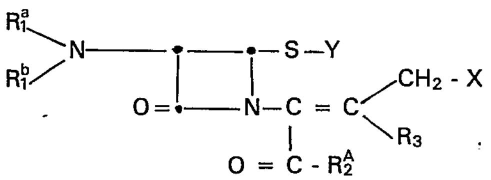

(I),

worin $R_1^a$ eine Aminoschutzgruppe und $R_1^b$ Wasserstoff oder einen Acylrest Ac bedeuten, oder worin $R_1^a$ und $R_1^b$ zusammen einen bivalenten Acylrest bilden, $R_2^A$ eine verätherte Hydroxygruppe ist, $R_3$ Methyl, Hydroxy, veräthertes oder verestertes Hydroxy, Cyano, tertiäres Amino oder Nitro ist, X Wasserstoff oder Halogen darstellt, und Y eine Gruppe $-SR_4$ bzw. $-SO_2-R_5$ ist, worin $R_4$ einen gegebenenfalls substituierten Heteroarylrest oder den Acylrest einer organischen Carbon- oder Thiocarbonsäure und $R_5$ einen gegebenenfalls substituierten Kohlenwasserstoffrest bedeutet, oder, wenn $R_3$ Hydroxy oder Methyl ist, Tautomeren davon, dadurch gekennzeichnet, dass man eine Verbindung der Formel

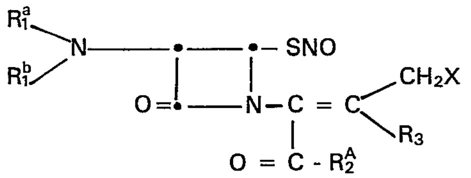

(III)

oder, wenn $R_3$ Hydroxy oder Methyl ist, ein Tautomeres davon mit einem Nitrosierungsmittel und einer Verbindung der Formel H-Y (IV) behandelt.

2. Verfahren nach Patentanspruch 1, dadurch gekennzeichnet, dass man Verbindungen der Formel I, worin $R_1^a$ eine Acylgruppe der Formel

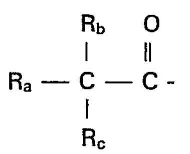

(IA)

darstellt, worin $R_a$ unsubstituiertes oder durch geschütztes Hydroxy, z.B. tert.-Butoxycarbonyloxy oder 2-Tetrahydropyranyloxy, Niederalkoxy, z.B. Methoxy, oder geschütztes Aminomethyl, z.B. tert.-Butoxycarbonylaminomethyl, substituiertes Phenyl oder Phenyloxy, oder Thienyl, z.B. 2-Thienyl, Furyl, z.B. 2-Furyl, Cyclohexadienyl, z.B. 1,4-Cyclohexadienyl, oder geschütztes 3-Amino-3-carboxypropyl, z.B. 3-Benzoylamino-3-diphenylmethoxycarbonyl-propyl, und $R_b$ und $R_c$ Wasserstoff sind, und $R_1^b$ Wasserstoff darstellt, $R_2^A$ Niederalkoxy, z.B. Methoxy oder tert.-Butoxy, 2-Halogenniederalkoxy, z.B. 2-Jod- oder 2,2,2-Trichloräthoxy, 2-Niederalkenyloxy, z.B. Allyloxy, unsubstituiertes oder in 4-Stellung durch Nitro substituiertes Benzyloxy, unsubstituiertes oder in 4- und 4'-Stellung durch Methoxy disubstituiertes Diphenylmethoxy oder Trityloxy ist, $R_3$ Methyl, Niederalkoxy, z.B. Methoxy, Phenylniederalkoxy, z.B. Benzyloxy, Hydroxy, Sulfonyloxy, z.B. Methan- oder p-Toluolsulfonyloxy, Nitrito, Phosphinoyloxy, z.B. Diphenylphosphinoyloxy, Phosphoryloxy, z.B. Diäthoxyphosphoryloxy, Halogen, z.B. Brom, Cyano, Nitro, Diniederalkylamino, z.B. Dimethylamino, Dicycloalkylamino, z.B. Dicyclohexylamino, 1-Pyrrolidinyl, 4-Morpholinyl oder 4-Methyl-1-piperazinyl bedeutet, X Wasserstoff oder Halogen, z.B. Brom, ist, und Y für Heteroarylthio, wie Oxazolyl-, z.B. 1,3-Oxazol-2-yl-, Thiazolyl- z.B. 1,3-Thiazol-2-yl-, Benzoxazol-2-yl- oder Benzthiazol-2-ylthio, oder für unsubstituiertes oder durch Niederalkyl, z.B. Methyl, Niederalkoxy, z.B. Methoxy, Halogen, z.B. Brom, oder Nitro substituiertes Benzolsulfonyl steht, oder, wenn $R_3$ Hydroxy oder Methyl ist, Tautomere davon herstellt.

3. Verfahren nach einem der Patentansprüche 1 oder 2, dadurch gekennzeichnet, dass man Verbindungen der Formel I, worin $R_1^a$ für einen Acetylrest der Formel IA steht, worin $R_a$ Phenyl, Thienyl, z.B. 2-Thienyl, oder Phenyloxy ist, und $R_b$ und $R_c$ Wasserstoff bedeuten, $R_1^b$ Wasserstoff ist, $R_2^A$ Niederalkoxy, z.B. tert.-Butoxy, 2-Halogenniederalkoxy, z.B. 2,2,2-Trichloräthoxy, gegebenenfalls in 4-Stellung durch Nitro substituiertes Benzyloxy, oder Diphenylmethoxy ist, X Wasserstoff oder Halogen, z.B. Brom, darstellt, $R_3$ Methyl, Hydroxy, Niederalkoxy, z.B. Methoxy, Sulfonyloxy, z.B. Methansulfonyloxy, Nitrito, Diniederalkylamino, z.B. Dimethylamino, 1-Pyrrolidinyl oder 1-Morpholinyl bedeutet und Y bicyclisches Heteroarylthio, z.B. Benzthiazol-2-ylthio, oder unsubstituiertes oder in 4-Stellung durch Niederalkyl, z.B. Methyl, substituiertes Benzolsulfonyl darstellt, oder, wenn $R_3$ Hydroxy oder Methyl ist, Tautomere davon herstellt.

4. Verfahren nach Patentanspruch 1, dadurch gekennzeichnet, dass man den 3-Methyl-2-[4-(4-methylphenylsulfonylthio)-2-oxo-3-phenoxyacetylamino-azetidin-1-yl]-3-butensäure-diphenylmethylester herstellt.

5. Verfahren nach Patentanspruch 1, dadurch gekennzeichnet, dass man den 2-[4-(Benzthiazol-2-yldithio)-2-oxo-3-phenoxyacetylamino-azetidin-1-yl]-3-methyl-3-butensäure-diphenylmethylester herstellt.

6. Verfahren nach Patentanspruch 1, dadurch gekennzeichnet, dass man den 3-Methyl-2-[4-(4-methylphenylsulfonylthio)-2-oxo-3-phenylacetylamino-azetidin-1-yl]-3-butensäure-diphenylmethylester herstellt.

7. Verfahren nach Patentanspruch 1, dadurch gekennzeichnet, dass man den 2-[4-(Benzthiazol-2-yldithio)-2-oxo-3-phenylacetylamino-azetidin-1-yl]-3-methyl-3-butensäure-diphenylmethylester herstellt.

8. Verfahren nach Patentanspruch 1, dadurch gekennzeichnet, dass man die Ausgangsverbindung der Formel III in situ herstellt, indem man ein Mercapto-azetidinon der Formel

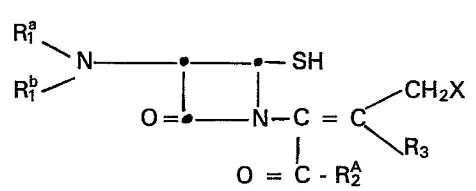

(II)

oder ein Salz oder, wenn $R_3$ Hydroxy oder Methyl ist, ein Tautomeres davon, mit einem Nitrosierungsmittel behandelt, und ohne Isolierung aus dem Reaktionsgemisch weiter zu den Verbindungen der Formel I umsetzt.

9. Verfahren nach Patentanspruch 1 zur Herstellung von Verbindungen der Formel I, worin $R_1^a$ einen Acylrest Ac und $R_1^b$ Wasserstoff bedeutet und $R_2^A$, $R_3$ und Y die in Patentanspruch 1 angegebenen Bedeutungen haben, dadurch gekennzeichnet, dass man die Ausgangsverbindung der Formel III, worin $R_1^a$ einen Acylrest Ac und $R_1^b$ Wasserstoff bedeutet und $R_2^A$, $R_3$ und X die im Patentanspruch 1 angegebenen Bedeutungen haben, in situ herstellt, indem man ein Diazabicyclohepten der Formel

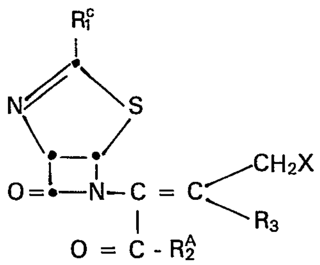

(V)

worin $R_1^c$ einen von der Acylgruppe Ac abgeleiteten, um die Carbonylgruppe verminderten Rest bedeutet, oder, wenn $R_3$ Hydroxy oder Methyl ist, ein Tautomeres davon, mit einem sauren Hydrolysemittel behandelt und ein erhaltenes Zwischenprodukt der Formel

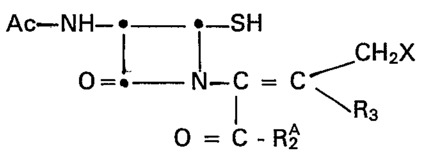

(II')

oder, wenn $R_3$ Hydroxy oder Methyl ist, ein Tautomeres davon, gegebenenfalls ohne Isolierung aus dem Reaktionsgemisch mit einem Nitrosierungsmittel behandelt, und ohne Isolierung aus dem Reaktionsgemisch weiter zu den Verbindungen der Formel I umsetzt.

10. Verfahren nach Patentanspruch 1 zur Herstellung von Verbindungen der Formel

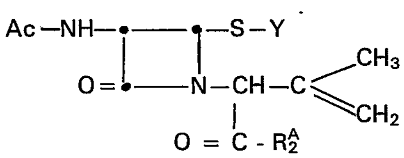

(I'),

dadurch gekennzeichnet, dass man die Ausgangsverbindung der Formel

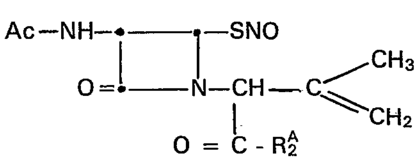

(III'')

in situ herstellt, indem man ein Penicillin-1-oxid der Formel

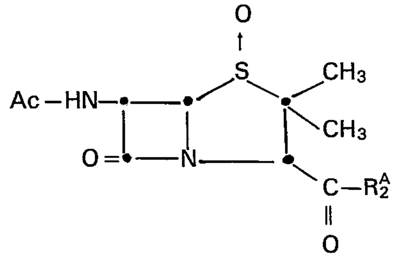

(VI)

mit einer Verbindung des 3wertigen Phosphors umsetzt, ein erhaltenes Zwischenprodukt der Formel

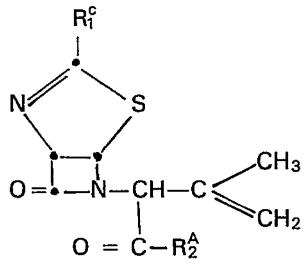

(V'),

worin $R_1^c$ einen von der Acylgruppe Ac abgeleiteten, um die Carbonylgruppe verminderten Rest bedeutet, gegebenenfalls ohne Isolierung aus dem Reaktionsgemisch mit einem sauren Hydrolysemittel behandelt und das erhaltene Zwischenprodukt der Formel

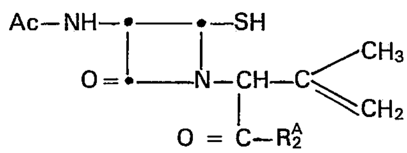

(II'')

gegebenenfalls ohne Isolierung aus dem Reaktionsgemisch mit einem Nitrosierungsmittel behandelt, und ohne Isolierung aus dem Reaktionsgemisch weiter zu den Verbindungen der Formel I umsetzt.

11. Verfahren nach einem der Patentansprüche 1 bis 10, dadurch gekennzeichnet, dass man als Nitrosierungsmittel einen Niederalkylester der salpetrigen Säure verwendet.

12. Verfahren nach einem der Patentansprüche 9 oder 10, dadurch gekennzeichnet, dass man als saures Hydrolysemittel eine anorganische Säure oder eine organische Sulfonsäure verwendet.

13. Verfahren nach Patentanspruch 10, dadurch gekennzeichnet, dass man als Verbindung des dreiwertigen Phosphors ein Triniederalkylphosphit verwendet.

14. Verfahren nach einem der Patentansprüche 1 bis 10, dadurch gekennzeichnet, dass man die Nitrosierungsreaktion und die Umsetzung mit den Verbindungen der Formel IV in einem inerten Lösungsmittel oder Lösungsmittelgemisch, z.B. im System Wasser-Methylenchlorid, bei —70° bis +50°C durchführt.

15. Verfahren nach einem der Patentansprüche 9 oder 10, dadurch gekennzeichnet, dass man die Hydrolyse der Diazabicycloheptene der Formel V und V' in Wasser, in einem organischen Lösungsmittel oder in einem Lösungsmittelgemisch, z.B. im System Wasser-Methylenchlorid, bei 10° bis 30°C durchführt.

16. Verfahren nach Patentanspruch 10, dadurch gekennzeichnet, dass man die Überführung der Penicillin-1-oxide der Formel VI in die bicyclischen Amide der Formel V' in einem inerten Lösungsmittel, z.B. in Äthylenchlorid, bei 70° bis 110°C ausführt.

17. Verfahren nach einem der Patentansprüche 9 oder 10, dadurch gekennzeichnet, dass man die Reaktionsschritte ohne Isolierung der verfahrensgemäss erhältlichen Zwischenprodukte durchführt.

18. 4-Nitrosothio-azetidinone der Formel

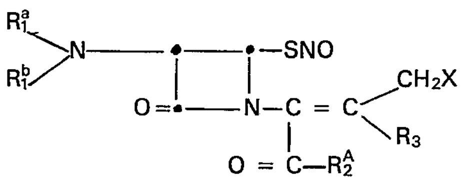

worin $R_1^a$ eine Aminoschutzgruppe und $R_1^b$ Wasserstoff oder einen Acylrest Ac bedeuten, oder worin $R_1^a$ und $R_1^b$ zusammen einen bivalenten Acylrest bilden, $R_2^A$ eine verätherte Hydroxygruppe ist, $R_3$ Methyl, Hydroxy, veräthertes oder verestertes Hydroxy, Cyano, tertiäres Amino oder Nitro ist und X Wasserstoff oder Halogen darstellt, oder, wenn $R_3$ Hydroxy oder Methyl ist, Tautomere davon.

19. 3-Methyl-2-(4-nitrosothio-2-oxo-3-phenoxyacetylamino-azetidin-1-yl)-3-butensäure-diphenylmethylester gemäss Patentanspruch 18.

20. 3-Methyl-2-(4-nitrosothio-2-oxo-3-phenylacetylamino-azetidin-1-yl)-3-butensäure-diphenylmethylester gemäss Patentanspruch 18.

21. Verfahren zur Herstellung von 4-Nitrosothio-azetidinonen der Formel

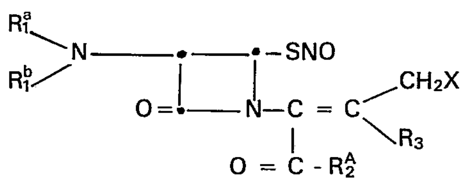

(III)

worin $R_1^a$ eine Aminoschutzgruppe und $R_1^b$ Wasserstoff oder einen Acylrest Ac bedeuten, oder worin $R_1^a$ und $R_1^b$ zusammen einen bivalenten Acylrest bilden, $R_2^A$ eine verätherte Hydroxygruppe ist, $R_3$ Methyl, Hydroxy, veräthertes oder verestertes Hydroxy, Cyano, tertiäres Amino oder Nitro ist und X Wasserstoff oder Halogen darstellt, oder, wenn $R_3$ Hydroxy oder Methyl ist, Tautomeren davon, dadurch gekennzeichnet, dass man

a) ein Mercapto-azetidinon der Formel

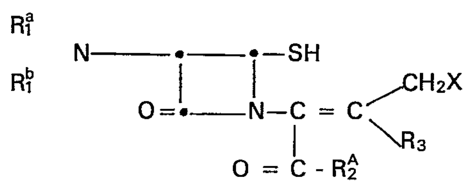

(II)

oder ein Salz oder, wenn $R_3$ Hydroxy oder Methyl ist, ein Tautomeres davon, mit einem Nitrosierungsmittel behandelt, oder dass man

b) zur Herstellung von 4-Nitrosothio-azetidinonen der Formel

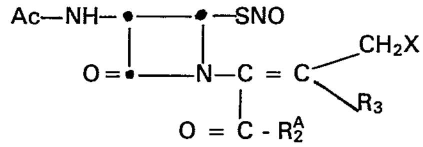

(III'),

worin Ac eine Acylgruppe bedeutet, und $R_2^A$, $R_3$ und X die unter Formel III angegebenen Bedeutungen haben, oder, wenn $R_3$ Hydroxy oder Methyl ist, einem Tautomeren davon, ein Diazabicyclohepten der Formel

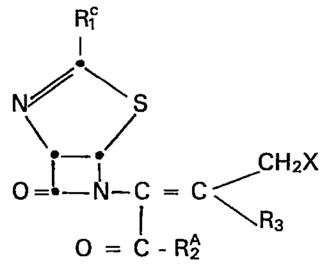

(V)

worin $R_1^c$ einen von der Acylgruppe Ac abgeleiteten, um die Carbonylgruppe verminderten Rest bedeutet, oder, wenn $R_3$ Hydroxy oder Methyl ist, ein Tautomeres davon, mit einem sauren Hydrolysemittel behandelt und ein erhaltenes Zwischenprodukt der Formel

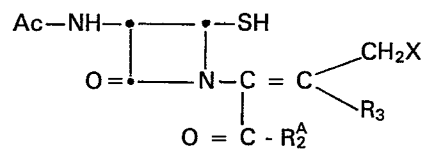

(II')

oder, wenn $R_3$ Hydroxy oder Methyl ist, ein Tautomeres davon, gegebenenfalls ohne Isolierung aus dem Reaktionsgemisch mit einem Nitrosierungsmittel behandelt, oder dass man

c) zur Herstellung von 4-Nitrosothio-azetidinonen der Formel

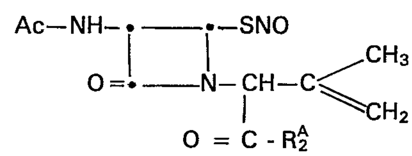

(III'')

ein Penicillin-1-oxid der Formel

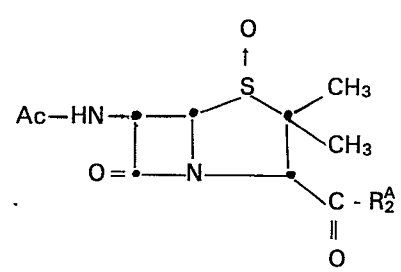

(VI)

mit einer Verbindung des 3wertigen Phosphors umsetzt, ein erhaltenes Zwischenprodukt der Formel

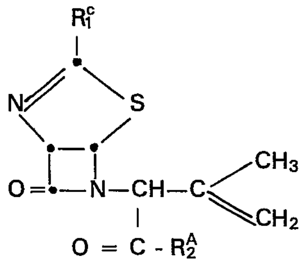

(V'),

worin $R_1^c$ die unter Formel V angegebene Bedeutung hat, gegebenenfalls ohne Isolierung aus dem Reaktionsgemisch mit einem sauren Hydrolysemittel behandelt und das erhaltene Zwischenprodukt der Formel

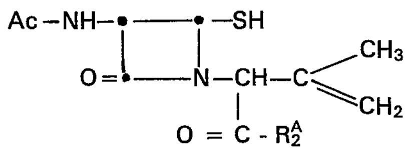

(II'')

gegebenenfalls ohne Isolierung aus dem Reaktionsgemisch mit einem Nitrosierungsmittel behandelt.

**Claims**

1. A process for the preparation of a 4-thioazetidinone compound of the formula

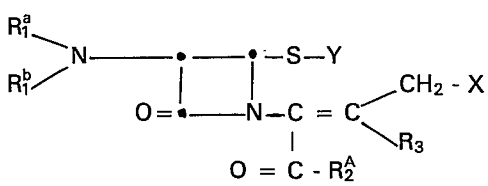

(I),

in which $R_1^a$ represents a protecting amino group and $R_1^b$ represents hydrogen or an acyl radical Ac, or in which $R_1^a$ and $R_1^b$ together represent a bivalent acyl radical, $R_2^A$ represents an etherified hydroxyl group, $R_3$ represents methyl, hydroxy, etherified or esterified hydroxy, cyano, tertiary amino or nitro, X represents hydrogen or halogen, and Y represents an $-SR_4$ or $-SO_2-R_5$ group, in which formula $R_4$ represents an unsubstituted or substituted heteroaryl radical or the acyl radical of an organic carboxylic or thiocarboxylic acid and $R_5$ represents an unsubstituted or substituted hydrocarbon radical, or, if $R_3$ represents hydroxy or methyl, a tautomer thereof, which process comprises treating a compound of the formula

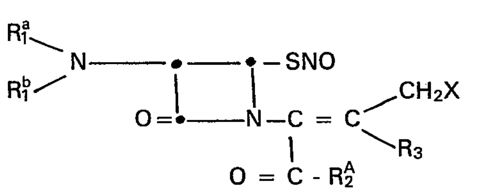

(III)

or, if $R_3$ represents hydroxy or methyl, a tautomer thereof, with a nitrosating agent and a compound of the formula H - Y (IV).

2. A process according to claim 1, which comprises preparing a compound of the formula I, in which $R_1^a$ represents an acyl group of the formula

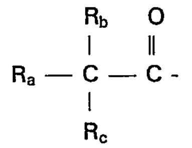

(IA)

in which $R_a$ represents phenyl or phenyloxy each of which is unsubstituted or substituted by protected hydroxy, for example tert-butoxycarbonyloxy or 2-tetrahydropyranyloxy, by lower alkoxy, for example methoxy, or by protected aminomethyl, for example tert-butoxycarbonylaminomethyl; or $R_a$ represents thienyl, for example 2-thienyl; furyl, for example 2-furyl; cyclohexadienyl, for example 1,4-cyclohexadienyl; or protected 3-amino-3-carboxypropyl, for example 3-benzoylamino-3-diphenylmethoxycarbonylpropyl, and $R_b$ and $R_c$ represent hydrogen, and $R_1^b$ represents hydrogen, $R_2^A$ represents lower alkoxy, for example methoxy or tert-butoxy; 2-halo-lower alkoxy, for example 2-iodoethoxy or 2,2,2-trichloro-ethoxy; 2-lower alkenyloxy, for example allyloxy; benzyloxy that is unsubstituted or substituted in the 4-position by nitro; diphenylmethoxy that is unsubstituted or disubstituted in the 4- and 4'-position by methoxy; or trityloxy, $R_3$ represents methyl, lower alkoxy, for example methoxy, phenyl-lower alkoxy, for example benzyloxy, hydroxy, sulphonyloxy, for example methanesulphonyloxy or p-toluenesulphonyloxy, nitrito, phosphinoyloxy, for example diphenylphosphinoyloxy, phosphoryloxy, for example diethoxyphosphoryloxy, halogen, for example bromine, cyano, nitro, di-lower alkylamino, for example dimethylamino, dicycloalkylamino, for example dicyclohexylamino, pyrrolidin-1-yl, morpholin-4-yl or 4-methylpiperazin-1-yl, X represents hydrogen or halogen, for example bromine, and Y represents heteroarylthio, such as oxazolylthio, for example 1,3-oxazol-2-ylthio, thiazolylthio, for example 1,3-thiazol-2-ylthio, benzoxazol-2-ylthio or benzthiazol-2-ylthio or benzenesulphonyl that is unsubstituted or substituted by lower alkyl, for example methyl, lower alkoxy, for example methoxy, halogen, for example bromine, or by nitro, or, if $R_3$ represents hydroxy or methyl, a tautomer thereof.

3. A process according to either claim 1 or claim 2, which comprises preparing a compound of the formula I, in which $R_1^a$ represents an acetyl radical of the formula IA, in which $R_a$ represents phenyl, thienyl, for example 2-thienyl, or phenyloxy, and $R_b$ and $R_c$ represent hydrogen, $R_1^b$ represents hydrogen, $R_2^A$ represents lower alkoxy, for example tert-butoxy, 2-halo-lower alkoxy, for example 2,2,2-trichloroethoxy, benzyloxy which is unsubstituted or substituted in the 4-position by nitro, or $R_2^A$ represents diphenylmethoxy, X represents hydrogen or halogen, for example bromine, $R_3$ represents methyl, hydroxy, lower alkoxy, for example methoxy, sulphonyloxy, for example methanesulphonyloxy, nitrito, di-lower alkylamino, for example dimethylamino, pyrrolidin-1-yl or morpholin-1-yl, and Y represents bicyclic heteroarylthio, for example benzthiazol-2-ylthio, or benzenesulphonyl, that is unsubstituted or substituted in the 4-position by lower alkyl, for example methyl, or, if $R_3$ represents hydroxy or methyl, a tautomer thereof.

4. A process according to claim 1, which comprises preparing 3-methyl-2-[4-(4-methylphenylsulphonylthio)-2-oxo-3-phenoxyacetylaminoazetidin-1-yl]-3-butenoic acid diphenylmethyl ester.

5. A process according to claim 1. which comprises preparing 2-[4-(benzthiazol-2-yldithio)-2-oxo-3-phenoxyacetylaminoazetidin-1-yl]-3-methyl-3-butenoic acid diphenylmethyl ester.

6. A process according to claim 1, which comprises preparing 3-methyl-2-[4-(4-methylphenylsulphonylthio)-2-oxo-3-phenylacetylaminoazetidin-1-yl]-3-butenoic acid diphenylmethyl ester.

7. A process according to claim 1, which comprises preparing 2-[4-(benzthiazol-2-yldithio)-2-oxo-3-phenylacetylaminoazetidin-1-yl]-3-methyl-3-butenoic acid diphenylmethyl ester.

8. A process according to claim 1, which comprises preparing in situ the starting compound of the formula III by treating a mercaptoazetidinone of the formula

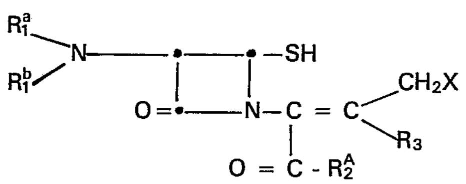

(II)

or a salt thereof, or, if $R_3$ represents hydroxy or methyl, a tautomer thereof, with a nitrosating agent, and further reacting, without isolating the nitroso compound from the reaction mixture, to form a compound of the formula I.

9. A process according to claim 1 for the preparation of a compound of the formula I in which $R_1^a$ represents an acyl radical Ac and $R_1^b$ represents hydrogen and $R_2^A$, $R_3$ and Y are as defined in claim 1, which process comprises preparing in situ the starting compound of the formula III, in which $R_1^a$ represents an acyl radical Ac and $R_1^b$ represents hydrogen and $R_2^A$, $R_3$ and X are as defined in claim 1, by treating a diazabicycloheptene of the formula

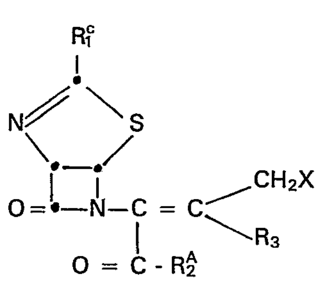

(V)

in which $R_1^c$ represents a radical which is derived from the acyl group Ac and from which the carbonyl group has been removed, or, if $R_3$ represents hydroxy or methyl, a tautomer thereof, with an acidic hydrolysis agent, and treating a resultant intermediate of the formula

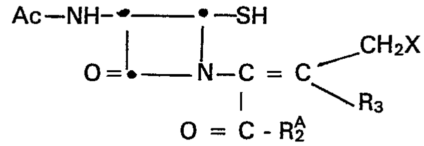

(II')

or, if $R_3$ represents hydroxy or methyl, a tautomer thereof, with or without isolating said intermediate from the reaction mixture, with a nitrosating agent, and further reacting, without isolating the nitroso compound from the reaction mixture, to form a compound of the formula I.

10. A process according to claim 1 for the preparation of a compound of the formula

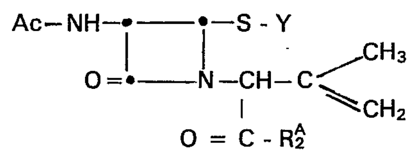

(I'),

which process comprises preparing in situ the starting compound of the formula

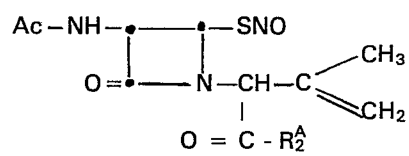

(III'')

by reacting a penicillin-1-oxide of the formula

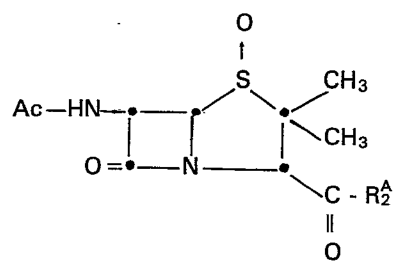

(VI)

with a compound of trivalent phosphorus, treating a resultant intermediate of the formula

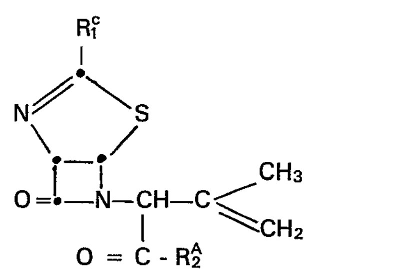

(V'),

in which $R_1^c$ represents a radical which is derived from the acyl group Ac and from which the carbonyl group has been removed, with or without isolating said intermediate from the reaction mixture, with an acidic hydrolysing agent, and treating the resultant intermediate of the formula

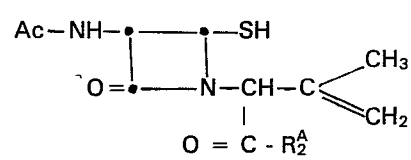

(II'')

with or without isolating said intermediate from the reaction mixture, with a nitrosating agent, and further reacting, without isolating the nitroso compound from the reaction mixture, to form a compound of the formula I.

11. A process according to any one of claims 1 to 10, wherein the nitrosating agent is a lower alkyl ester of nitrous acid.

12. A process according to either claim 9 or claim 10, wherein the acidic hydrolysing agent is an inorganic acid or an organic sulphonic acid.

13. A process according to claim 10, wherein the compound of trivalent phosphorus is a tri-lower alkyl phosphite.

14. A process according to any one of claims 1 to 10, which comprises carrying out the nitrosation reaction and the reaction with the compound of the formula IV in an inert solvent or solvent mixture, for example in the system water/methylene chloride, in the temperature range from —70° to +50°C.

15. A process according to either claim 9 or claim 10, which comprises carrying out the hydrolysis of the diazabicycloheptenes of the formulae V and V' in water, in an organic solvent or in a solvent mixture, for example in the system water/methylene chloride, in the temperature range from 10° to 30°C.

16. A process according to claim 10, which comprises carrying out the conversion of the penicillin-1-oxide of the formula VI into the bicyclic amide of the formula V' in an inert solvent, for example in ethylene chloride, in the temperature range from 70° to 110°C.

17. A process according to either claim 9 or claim 10, which comprises carrying out the reaction steps without isolating the intermediates obtainable according to the process.

18. A 4-nitrosothioazetidinone of the formula

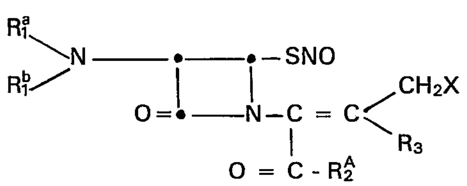

(III)

in which $R_1^a$ represents a protecting amino group and $R_1^b$ represents hydrogen or an acyl radical Ac, or in which $R_1^a$ and $R_1^b$ together represent a bivalent acyl radical, $R_2^A$ represents an etherified hydroxyl group $R_3$ represents methyl, hydroxy, etherified or esterified hydroxy, cyano, tertiary amino or nitro, and X represents hydrogen or halogen, or, if $R_3$ represents hydroxy or methyl, a tautomer thereof.

19. 3-Methyl-2-(4-nitrosothio-2-oxo-3-phenoxyacetylamino-azetidin-1-yl)-3-butenoic acid diphenylmethyl ester according to claim 18.

20. 3-Methyl-2-(4-nitrosothio-2-oxo-3-phenylacetylamino-azetidin-1-yl)-3-butenoic acid diphenylmethyl ester according to claim 18.

21. A process for the preparation of a 4-nitrosothioazetidinone of the formula

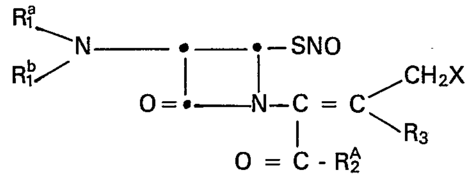

(III)

in which $R_1^a$ represents a protecting amino group and $R_1^b$ represents hydrogen or an acyl radical Ac, or in which $R_1^a$ and $R_1^b$ together represent a bivalent acyl radical, $R_2^A$ represents an etherified hydroxyl group $R_3$ represents methyl, hydroxy, etherified or esterified hydroxy, cyano, tertiary amino or nitro, and X represents hydrogen or halogen, or, if $R_3$ represents hydroxy or methyl, a tautomer thereof, which process comprises

a) treating a mercaptoazetidinone of the formula

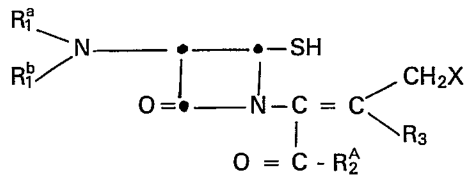

(II)

or a salt thereof, or, if $R_3$ represents hydroxy or methyl, a tautomer thereof, with a nitrosating agent, or

b) to prepare a 4-nitrosothioazetidinone of the formula

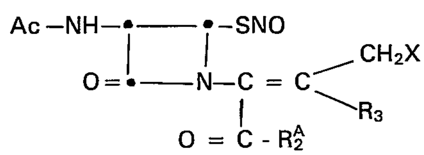

(III'),

in which Ac represents an acyl group, and $R_2^A$, $R_3$ and X are as defined for formula III, or, if $R_3$ represents hydroxy or methyl, a tautomer thereof, treating a diazabicycloheptene of the formula

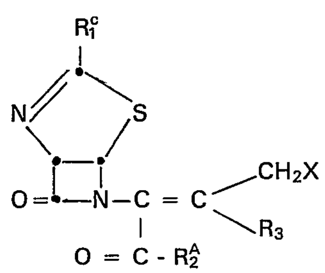

(V)

in which $R_1^c$ represents a radical which is derived from the acyl group Ac and from which the carbonyl group has been removed, or; if $R_3$ represents hydroxy or methyl, a tautomer thereof, with an acidic hydrolysis agent and treating a resultant intermediate of the formula

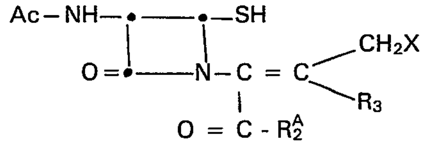

(II')

or, if $R_3$ represents hydroxy or methyl, a tautomer thereof, with or without isolating said intermediate from the reaction mixture, with a nitrosating agent, or

c) to prepare a 4-nitrosothioazetidinone of the formula

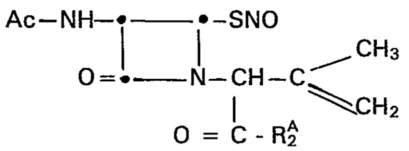

(III''),

reacting a penicillin-1-oxide of the formula

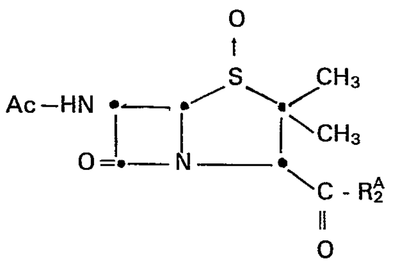

(VI)

with a compound of trivalent phosphorus, treating a resultant intermediate of the formula

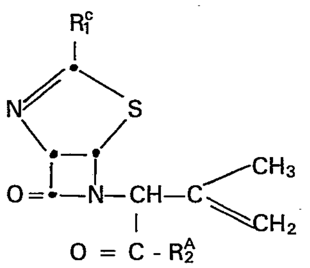

(V'),

in which $R_1^c$ is as defined for formula V, with or without isolating said intermediate from the reaction mixture, with an acidic hydrolysing agent, and treating the resultant intermediate of the formula

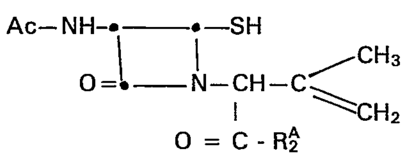

(II'')

with or without isolating said intermediate from the reaction mixture, with a nitrosating agent.

**Revendications**

1. Procédé de préparation de dérivés de la 4--thioazétidinone de formule:

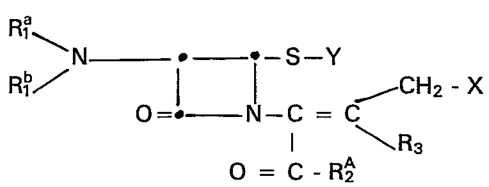

I

dans laquelle $R_1^a$ représente un groupe protecteur du groupe amino et $R_1^b$ représente l'hydrogène ou un radical acyle Ac, ou bien $R_1^a$ et $R_1^b$ forment ensemble un radical acyle bivalent, $R_2^A$ représente un groupe hydroxy éthérifié, $R_3$ représente un groupe méthyle, hydroxy, hydroxy éthérifié ou estérifié, cyano, tert.--amino ou nitro, X représente l'hydrogène ou un halogène et Y représente un groupe $-SR_4$ ou $-SO_2-R_5$ dans lequel $R_4$ représente un reste hétéroaryle éventuellement substitué ou le radical acyle d'un acide organique carboxylique ou thiocarboxylique et $R_5$ représente un reste hydrocarboné éventuellement substitué, ou bien, lorsque $R_3$ représente un groupe hydroxy ou méthyle, les tautomères de ces composés, caractérisé en ce que l'on traite un composé de formule:

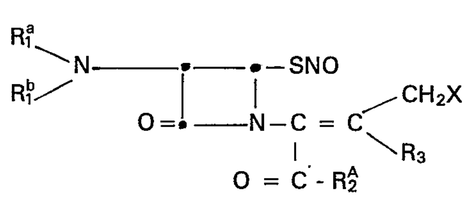

(III)

ou bien, lorsque $R_3$ représente un groupe hydroxy ou méthyle, un tautomère de ce composé, par un agent nitrosant et un composé de formule H - Y (IV).

2. Procédé selon la revendication 1, caractérisé en ce que l'on prépare des composés de formule I dans laquelle $R_1^a$ représente un radical acyle de formule:

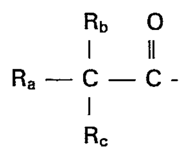

(IA)

dans laquelle $R_a$ représente un groupe phényle ou phényloxy non substitué ou substitué par un groupe hydroxy protégé, par exemple tert.-butoxycarbonyloxy ou 2-tétrahydropyrannyloxy, alcoxy inférieur, par exemple méthoxy, ou aminométhyle protégé, par exemple tert.-butoxycarbonylaminométhyle, ou un groupe thiényle, par exemple 2-thiényle, furyle, par exemple 2-furyle, cyclohexadiényle, par exemple 1,4-cyclohexadiényle, ou 3-amino-3-carboxypropyle protégé, par exemple 3-benzoylamino--3-diphénylméthoxycarbonylpropyle, et $R_b$ et $R_c$ représentent l'hydrogène, et $R_1^b$ représente l'hydrogène, $R_2^A$ représente un groupe alcoxy inférieur, par exemple méthoxy ou tert.-butoxy, 2-halogénoalcoxy inférieur, par exemple 2-iodo- ou 2,2,2-trichloroéthoxy, 2-alcényloxy inférieur, par exemple allyloxy, benzyloxy non substitué ou substitué par un groupe nitro en position 4, diphénylméthoxy ou trityloxy non substitué ou disubstitué dans les positions 4 et 4' par des groupes méthoxy, $R_3$ représente un groupe méthyle, alcoxy inférieur, par exemple méthoxy, phénylalcoxy inférieur, par exemple benzyloxy, hydroxy, sulfonyloxy, par exemple méthane- ou p-toluènesulfonyloxy, nitrito, phosphinoyloxy, par exemple diphénylphosphinoyloxy, phosphoryloxy, par exemple diéthoxyphosphoryloxy, un halogène, par exemple le brome, un groupe

cyano, nitro, di-(alkyle inférieur)-amino, par exemple diméthylamino, dicycloalkylamino, par exemple dicyclohexylamino, 1-pyrrolidinyle, 4-morpholinyle ou 4-méthyle-1-pipérazinyle, X représente l'hydrogène ou un halogène, par exemple le brome, et Y représente un groupe hétéroarylthio tel que oxazolyl-, par exemple 1,3-oxazole-2-yl, thiazolyl-, par exemple 1,3-thiazole-2-yl-, benzoxazole-2-yl- ou benzothiazole-2-ylthio, ou un groupe benzène-sulfonyle non substitué ou substitué par un groupe alkyle inférieur, par exemple méthyle, alcoxy inférieur, par exemple méthoxy, un halogène, par exemple le brome, ou un groupe nitro, ou bien lorsque $R_3$ représente un groupe hydroxy ou méthyle, des tautomères de ces composés.

3. Procédé selon l'une des revendications 1 ou 2, caractérisé en ce que l'on prépare des composés de formule I dans laquelle $R_1^a$ représente un radical acétyle de formule IA dans laquelle $R_a$ représente un groupe phényle, thiényle, par exemple 2-thiényle, ou phényloxy, et $R_b$ et $R_c$ représentent l'hydrogène, $R_1^b$ représente l'hydrogène, $R_2^A$ représente un groupe alcoxy inférieur, par exemple tert.-butoxy, 2-halogénoalcoxy inférieur, par exemple 2,2,2-trichloroéthoxy, benzyloxy éventuellement substitué en position 4 par un groupe nitro, ou diphénylméthoxy, X représente l'hydrogène ou un halogène, par exemple le brome, $R_3$ représente un groupe méthyle, hydroxy, alcoxy inférieur, par exemple méthoxy, sulfonyloxy, par exemple méthanesulfonyloxy, nitrito, di-(alkyle inférieur)-amino, par exemple diméthylamino, 1-pyrrolidinyle ou 1-morpholinyle et Y représente un groupe hétéroarylthio bicyclique, par exemple benzothiazole-2-ylthio, ou benzènesulfonyle non substitué ou substitué en position 4 par un groupe alkyle inférieur, par exemple méthyle, ou bien, lorsque $R_3$ représente un groupe hydroxy ou méthyle, des tautomères de ces composés.

4. Procédé selon la revendication 1, caractérisé en ce que l'on prépare le 3-méthyl-2-[4-(4-méthylphénylsulfonylthio)-2-oxo-3-phénoxyacétylaminoazétidine-1-yl]-3-buténoate de diphénylméthyle.

5. Procédé selon la revendication 1, caractérisé en ce que l'on prépare le 2-[4-(benzothiazole-2-yldithio)-2-oxo-3-phénoxyacétylaminoazétidine-1-yl]-3-méthyl-3-buténoate de diphénylméthyle.

6. Procédé selon la revendication 1, caractérisé en ce que l'on prépare le 3-méthyl-2-[4-(4-méthylphénylsulfonylthio)-2-oxo-3-phénylacétylaminoazétidine-1-yl]-3-buténoate de diphénylméthyle.

7. Procédé selon la revendication 1, caractérisé en ce que l'on prépare le 2-[4-(benzothiazole-2-yldithio)-2-oxo-3-phénylacétylaminoazétidine-1-yl]-3-méthyl-3-buténoate de diphénylméthyle.

8. Procédé selon la revendication 1, caractérisé en ce que l'on prépare le composé de départ de formule III <u>in situ</u>, en traitant une mercapto-azétidinone de formule:

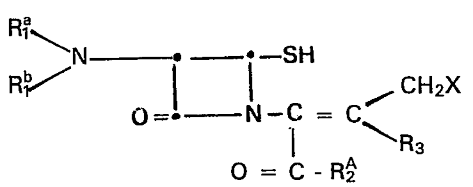

II

ou un sel d'un tel composé ou bien, lorsque $R_3$ représente un groupe hydroxy ou méthyle, un tautomère d'un tel composé, par un agent nitrosant, et on le convertit sans l'isoler du mélange de réaction en les composés de formule I.

9. Procédé selon la revendication 1, pour la préparation des composés de formule I dans laquelle $R_1^a$ représente un radical acyle Ac et $R_1^b$ représente l'hydrogène et $R_2^A$, $R_3$ et Y ont les significations indiquées dans la revendication 1, caractérisé en ce que l'on prépare <u>in situ</u> le composé de départ de formule III dans laquelle $R_1^a$ représente un radical acyle Ac et $R_1^b$ représente l'hydrogène, $R_2^A$, $R_3$ et X ayant les significations indiquées dans la revendication 1, en traitant un diazabicycloheptène de formule:

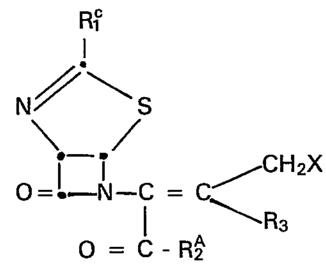

V

dans laquelle $R_1^c$ représente un reste dérivé du radical acyle Ac par élimination du groupe carbonyle, ou bien, lorsque R représente un groupe hydroxy ou méthyle, un tautomère de ce composé, par un agent hydrolysant acide, ce qui donne le produit intermédiaire de formule:

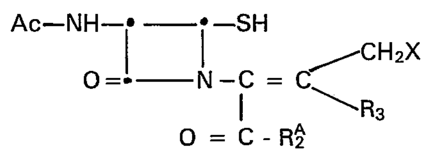

II'

ou bien, lorsque $R_3$ représente un groupe hydroxy ou méthyle, un tautomère de ce composé, qu'on traite, éventuellement sans l'isoler du mélange de réaction, par un agent nitrosant après quoi, toujours sans isoler du mélange de réaction, on convertit en les composés de formule I.

10. Procédé selon la revendication 1, pour préparer les composés de formule:

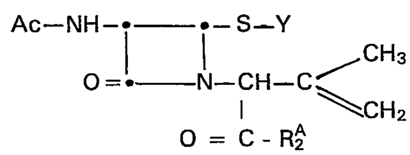

I'

caractérisé en ce que l'on prépare <u>in situ</u> le composé de départ de formule:

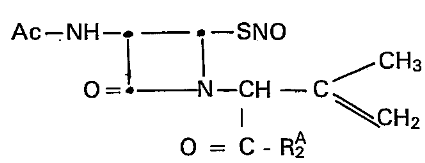

III''

en faisant réagir un 1-oxyde de pénicilline de formule:

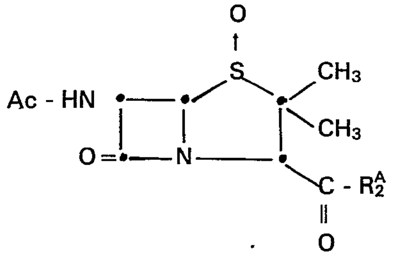

VI

avec un composé du phosphore trivalent, ce qui donne un produit intermédiaire de formule:

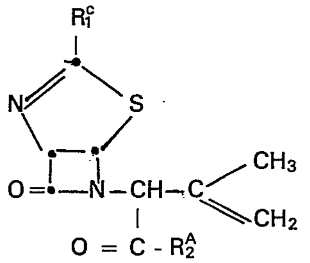

V'

dans laquelle $R_1^c$ représente un reste dérivé du radical acyle Ac par élimination du groupe carbonyle, qu'on traite, éventuellement sans l'isoler du mélange de réaction, par un agent hydrolysant acide, ce qui donne le produit intermédiaire de formule:

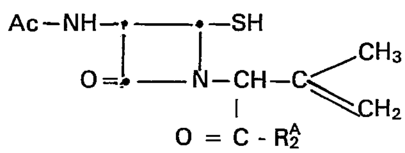

II''

qu'on traite, éventuellement sans l'isoler du mélange de réaction, par un agent nitrosant après quoi, sans l'isoler du mélange de réaction, on convertit en les composés de formule I.

11. Procédé selon l'une des revendications 1 à 10, caractérisé en ce que l'on utilise en tant qu'agent nitrosant un ester alkylique inférieur de l'acide nitreux.

12. Procédé selon l'une des revendications 9 ou 10, caractérisé en ce que l'on utilise en tant qu'agent hydrolysant acide un acide minéral ou un acide organique sulfonique.

13. Procédé selon la revendication 10, caractérisé en ce que l'on utilise en tant que composé du phosphore trivalent un phosphite de trialkyle inférieur.

14. Procédé selon l'une des revendications 1 à 10, caractérisé en ce que l'on effectue la réaction de nitrosation et la réaction avec les composés de formule IV dans un solvant ou mélange solvant inerte, par exemple dans le système eau-chlorure de méthylène, à une température de —70 à +50°C.

15. Procédé selon l'une des revendications 9 ou 10, caractérisé en ce que l'on effectue l'hydrolyse des diazabicycloheptènes de formules V et V' dans l'eau, dans un solvant organique ou dans un mélange solvant, par exemple le système eau-chlorure de méthylène, à une température de 10 à 30°C.

16. Procédé selon la revendication 10, caractérisé en ce que la conversion des 1-oxydes de pénicillines de formule VI en les amides bicycliques de formule V' est effectuée dans un solvant inerte, par exemple le chlorure d'éthylène, à une température de 70 à 110°C.

17. Procédé selon l'une des revendications 9 ou 10, caractérisé en ce que l'on réalise les stades de réaction sans isoler les produits intermédiaires obtenus éventuellement dans les opérations.

18. 4-nitrosothio-azétidinones de formule:

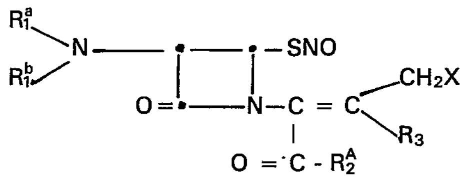

dans laquelle $R_1^a$ représente un groupe protecteur du groupe amino et $R_1^b$ représente l'hydrogène ou un radical acyle Ac, ou bien dans laquelle $R_1^a$ et $R_1^b$ forment ensemble un radical acyle bivalent, $R_2^A$ représente un groupe hydroxy éthérifié, $R_3$ représente un groupe méthyle, hydroxy, hydroxy éthérifié ou estérifié, cyano, tert.-amino ou nitro et X représente l'hydrogène ou un halogène, ou bien, lorsque $R_3$ représente un groupe hydroxy ou méthyle, leurs tautomères.

19. Le 3-méthyl-2-(4-nitrosothio-2-oxo-3-phénoxyacétylamino-azétidine-1-yl)-3-buténoate de diphénylméthyle selon la revendicationd 18.

20. Le 3-méthyl-2-(4-nitrosothio-2-oxo-3-phénylacétylamino-azétidine-1-yl)-3-buténoate de diphénylméthyle selon la revendication 18.

21. Procédé de préparation des 4-nitrosothio--azétidinones de formule:

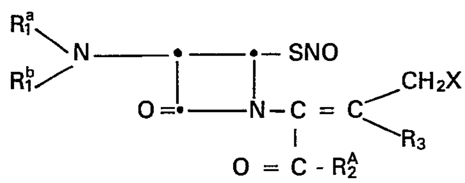

III

dans laquelle $R_1^a$ représente un groupe protecteur du groupe amino et $R_1^b$ représente l'hydrogène ou un radical acyle Ac, ou bien $R_1^a$ et $R_1^b$ forment ensemble un radical acyle bivalent, $R_2^A$ représente un groupe hydroxy éthérifié, $R_3$ représente un groupe méthyle, hydroxy, hydroxy éthérifié ou estérifié, cyano, tert.--amino ou nitro et X représente l'hydrogène ou un halogène, ou bien, lorsque $R_3$ représente un groupe hydroxy ou méthyle, de leurs tautomères, caractérisé en ce que:

a) on traite une mercapto-azétidinone de formule:

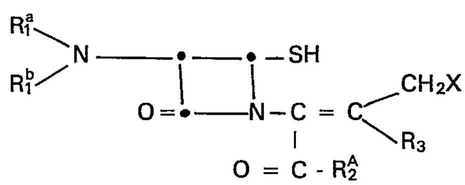

II

ou un sel de ce composé ou bien, lorsque $R_3$ représente un groupe hydroxy ou méthyle, un tautomère de ce composé, par un agent nitrosant, ou bien

b) pour préparer les 4-nitrosothio-azétidinones de formule:

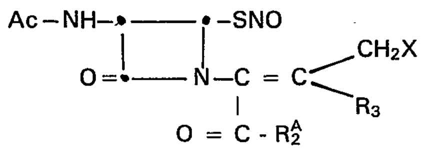

III'

dans laquelle Ac représente un radical acyle et $R_2^A$, $R_3$ et X ont les significations indiquées en référence à la formule III, ou bien, lorsque $R_3$ représente un groupe hydroxy ou méthyle, un tautomère de ces composés, on traite un diazabicycloheptène de formule:

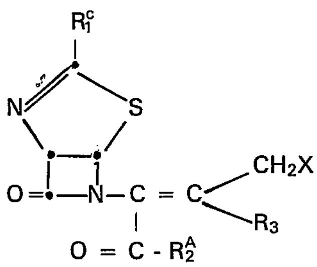

V

dans laquelle $R_1^c$ représente un reste dérivé du radical acyle Ac par élimination du groupe carbonyle, ou bien, lorsque $R_3$ représente un groupe hydroxy ou méthyle, un tautomère de ce composé, par un agent hydrolysant acide, ce qui donne un produit intermédiaire de formule:

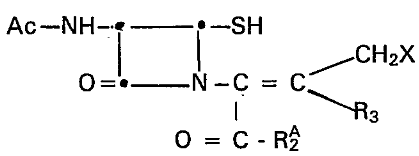

II'

ou bien, lorsque $R_3$ représente un groupe hydroxy ou méthyle, un tautomère d'un tel composé, qu'on traite, éventuellement sans l'isoler du mélange de réaction, par un agent nitrosant, ou bien

c) pour préparer les 4-nitrosothio-azétidinones de formule:

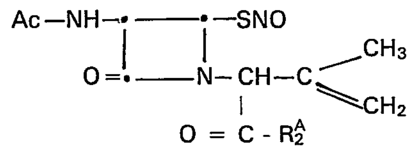

III''

on fait réagir un 1-oxyde de pénicilline de formule:

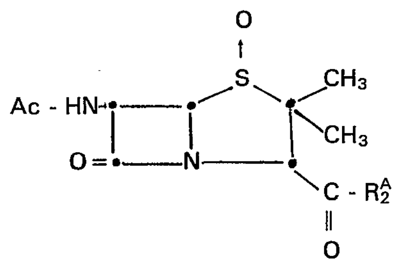

VI

avec un composé du phosphore trivalent, ce qui donne un produit intermédiaire de formule:

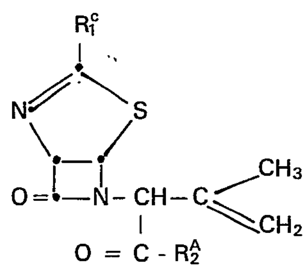

V'

dans laquelle $R_1^c$ a les significations indiquées en référence à la formule V, qu'on traite, éventuellement sans l'isoler du mélange de réaction, par un agent hydrolysant acide, ce qui donne le produit intermédiaire de formule:

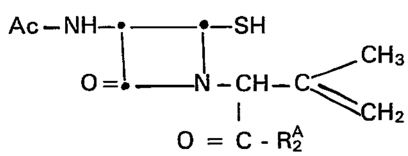

II''

qu'on traite, éventuellement sans l'isoler du mélange de réaction, par un agent nitrosant.